# EUROPEAN PATENT SPECIFICATION

(11) **EP 2 804 897 B1**
(45) Date of publication and mention of the grant of the patent: **13.07.2016**
(21) Application number: 13702700.9
(22) Date of filing: 18.01.2013
(51) Int. Cl.: C08K 3/00, C08K 5/00

(54) **ORAL PRODUCT**
ORALES PRODUKT
PRODUIT BUCCAL

(30) Priority: 20.01.2012 US 201261588904 P; 20.01.2012 US 201261588873 P; 20.01.2012 US 201261588851 P; 20.01.2012 US 201261588861 P; 31.10.2012 US 201261720852 P
(43) Date of publication of application: 26.11.2014
(73) Proprietor: Altria Client Services LLC, Richmond, Virginia 23230 (US)
(72) Inventor: GAO, Feng, Midlothian, VA 23114 (US); HULAN, Phillip, M., Midlothian, VA 23113 (US); GEE, Diane, Chesterfield, VA 23230 (US); FLORA, Jason, Richmond, VA 23235 (US); GRISCIK, Gregory, Midlothian, VA 23114 (US); ZHUANG, Shuzhong, Glen Allen, VA 23060 (US); ATCHLEY, Frank Scott, Tarpon Springs, Florida 34688 (US); DINOVI, Christopher, Joseph, Ruther Glen, VA 22546 (US)
(74) Representative: Wichmann, Hendrik
(86) International application number: PCT/US2013/022252
(87) International publication number: WO 2013/109961

(56) References cited:
- EP-A1- 2 226 171
- EP-A2- 0 118 972
- WO-A2-2008/133982

## Description

### TECHNICAL FIELD

This document relates to oral products including mouth-stable polymers and one or more additives.

### BACKGROUND

Tobacco can be enjoyed by adult tobacco consumers in a variety of forms. Smoking tobacco is combusted and the aerosol either tasted or inhaled (e.g., in a cigarette, cigar, or pipe). Smokeless tobacco products are not combusted and include: chewing tobacco, moist smokeless tobacco, snus, and dry snuff. Chewing tobacco is coarsely divided tobacco leaf that is typically packaged in a large pouch-like package and used in a plug or twist. Moist smokeless tobacco is a moist, more finely divided tobacco that is provided in loose form or in pouch form and is typically packaged in round cans and used as a pinch or in a pouch placed between an adult tobacco consumer's cheek and gum. Snus is a heat treated smokeless tobacco. Dry snuff is finely ground tobacco that is placed in the mouth or used nasally.

A growing number of governments are now implementing restrictions on smoking in public places, such as restaurants and transport facilities. In some countries, such as the United States, some workplaces are also covered by public restrictions. Smokeless products may also be banned by certain governments or workplaces.

Trans-buccal systems such as nicotine-containing chewing gum as well as transdermal nicotine delivery systems are well known in the art. These systems, however, do not consistently provide a suitable tobacco-like experience for some adult tobacco consumers.

Oral products providing flavor and/or one or more active ingredients are also known. One such oral product is chewing gum. Other oral products include hard candies (e.g., mints). Softer gelatin-based oral products are also known. Pharmaceutical and therapeutic products (e.g., cough-suppressant lozenges) can also be provided in a solid form for oral consumption. The flavor release and/or active agent release characteristics for an oral product are important for providing an improved consumer product.

EP2226171 A1 discloses a fiber composite which comprises fibers having an average fiber diameter of at most 30 nm and a matrix material and which has a haze of at most 5 according to JIS K 7136 when the fiber composite has a thickness of 100 µm. It further discloses a microfibrillated cellulose fiber dispersion which is a dispersion of fibers made of cellulose having cellulose type I crystal and having repeating units which may be substituted by a nicotinoyl group.

WO 2008/133982 A2 discloses an adhesive patch that comprises a medicament and an aversive agent, wherein the medicament can comprise nicotine, estrogen, nitroglycerin, lidocaine or fentanyl.

EP0118972 A2 discloses a filter for tobacco smoke which contains a fibrous ion exchange resin, wherein a layer containing said fibrous ion exchange resin can be inserted between other components of the filter perpendicular to the axis of the filter.

### SUMMARY

This specification describes an oral product that provides a satisfying tactile and/or flavor experience. In particular embodiments, the oral product can provide an extended additive release time. The oral product includes a body that is wholly receivable in an oral cavity of an adult consumer. The body includes a mouth-stable polymer matrix as defined in the claims, cellulosic fibers embedded in the stable polymer matrix, and one or more additives dispersed in the body such that it is released when the body is received within the oral cavity and exposed to saliva.

The oral product, according to certain embodiments, includes nicotine or a derivative thereof. The oral product can provide a tobacco-like flavor experience and favorable tactile experience. Combinations of additives (e.g., sweeteners, flavorants, and nicotine) can be combined to provide a favorable tactile and flavor experience.

The oral product, according to certain embodiments, includes flavorants, sweeteners, vitamins, minerals, therapeutic agents, nutraceuticals, energizing agents, soothing agents, coloring agents, amino acids, chemsthetic agents, antioxidants, food grade emulsifiers, pH modifiers, botanicals, teeth whitening agents, and/or alkaloids (e.g., caffeine). In some cases, the oral product can be substantially free of tobacco. Combinations of additives (e.g., sweeteners, flavorants, and caffeine) can be combined to provide a favorable tactile and flavor experience.

This specification also describes an oral tobacco product that provides a satisfying tactile and/or flavor experience. The oral tobacco product includes a body that is wholly receivable in an oral cavity of an adult tobacco consumer. The body includes a mouth-stable polymer matrix as defined in the claims and e.g. tobacco fibers embedded in the stable polymer matrix. In some embodiments, an oral product can include exhausted-tobacco fibers and one or more additives.

These and other embodiments can each optionally include one or more of the following features. In some embodiments, the oral product's body includes at least 10 weight percent of the mouth-stable polymer. The mouth-stable polymer matrix includes a polymer as defined in the claims, e.g. a polymer selected from the group consisting of polyurethane, silicon polymer, polyester, polyacrylate, polyethylene, poly(styrene-ethylene-butylene-styrene) ("SEBS"), poly(styrene-butadiene-styrene) ("SBS"), poly(styrene-isoprene-styrene)("SIS"), or any copolymer, mixture, or combination thereof. The oral product can also include a plasticizer dispersed in the mouth-stable polymer matrix. For example, the plasticizer can be propylene glycol, glycerin, vegetable oil, triglycerides, or a combination thereof. The oral product can also include a sweetener dispersed in the body. The sweetener can be saccharine, sucralose, aspartame, acesulfame potassium, or a combination thereof.

The oral product, according to certain embodiments, is substantially free of tobacco plant tissue. Nicotine added to the oral product can be either synthetic or derived from tobacco. In some embodiments, the oral product includes between 0.1 mg and 6 mg nicotine. The oral products can also include an additive selected from the group consisting of minerals, vitamins, dietary supplements, nutraceuticals, energizing agents, soothing agents, amino acids, chemsthetic agents, antioxidants, botanicals, teeth whitening agents, therapeutic agents, or a combination thereof. The nicotine and/or other additives can be absorbed into the cellulosic fibers and polymer matrix. The oral product, according to some embodiments, includes exhausted-tobacco fibers as the cellulosic fibers. In some embodiments, the oral product includes tobacco fibers.

The oral product's body can have at least 10 weight percent cellulosic fibers. The cellulosic fibers can be derived from plant tissue. The cellulosic fibers include cellulose or treated cellulose material. The cellulosic fibers can further include lignin and/or lipids. The cellulosic fibers can be non-tobacco cellulosic fibers. For example, the cellulosic fibers can be selected from the following: sugar beet fiber, wood pulp fiber, cotton fiber, bran fiber, citrus pulp fiber, grass fiber, willow fiber, poplar fiber, and combinations thereof. The cellulosic fibers may also be chemically treated prior to use. For example, the cellulosic fibers can be CMC, HPMC, HPC, or other treated cellulosic material.

The oral product can include flavorants. The flavorants can be natural or artificial. Flavorants can be selected from the following: licorice, wintergreen, cherry and berry type flavorants, Drambuie, bourbon, scotch, whiskey, spearmint, peppermint, lavender, cinnamon, cardamon, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, anise, coriander, coffee, mint oils from a species of the genus Mentha, cocoa, and combinations thereof. Synthetic flavorants can also be used. In certain embodiments, a combination of flavorants can be combined to imitate a tobacco flavor. The particular combination of flavorants can be selected from the flavorants that are generally recognized as safe ("GRAS"). Flavorants can also be included in the oral product as encapsulated flavorants.

The body of the oral product can have a variety of different shapes, some of which include disk, shield, rectangle, and square. According to certain embodiments, the body can have a length or width of between 5 mm and 25 mm and a thickness of between 1 mm and 10 mm.

The oral product's body can be compressible and springy. In some embodiments, the body has a compressibility @ 250 N of less than 95%, less than 90%, less than 85%, or less than 80%. In some embodiments, the body has a compressibility of @ 250 N of between 45% and 90%. The oral product's body can have a compressibility @ 425 N of less than 99%. For example, the body can have a compressibility @ 425 N of between 60% and 98%. The body can also have a percentage of springiness of at least 20%, at least 30%, at least 40%, at least 50%, at least 60%, at least 70%, or at least 75%. For example, the body can have a percentage of springiness of between 75% and 90%.

The oral product can also include an antioxidant. In some embodiments, the oral product includes between 0.01 weight percent and 5.0 weight percent antioxidant. Suitable antioxidants include ascorbyl palmitate, BHT, ascorbic acid, sodium ascorbate, monosterol citrate, tocopherols, propyl gallate, tertiary butylhydroquinone (TBHQ), butylated hydroxyanisole (BHA), Vitamin E, and derivatives thereof. The combination of antioxidant and nicotine can reduce the formation of nicotine-N-oxide.

The oral product can include a combination of soluble fibers and insoluble cellulosic fibers. In some embodiments, a ratio of soluble fiber to cellulosic fibers can be between 1:60 and 60:1. In some embodiments, the soluble fibers can include maltodextrin. In some embodiments, the soluble fibers comprise starch. The soluble fibers can be derived from com. In general, another aspect of the subject matter described in this specification is methods of making and using the oral product. The methods of making the oral product can include the actions of extruding a mouth-stable polymer having cellulosic fibers and/or one or more additives dispersed therein.

The details of one or more embodiments of the subject matter described in this specification are set forth in the accompanying drawings and the description below. Other features, aspects, and advantages of the subject matter will become apparent from the description, the drawings, and the claims.

### BRIEF DESCRIPTION OF THE DRAWINGS

Figure 1 is a perspective view of a pair of oral products.
Figures 2A-2O illustrate various exemplary shapes of oral products.
Figure 3A-3J illustrate oral products having various rod, stick, or tube configurations.
Figures 4A-4C are magnified pictures of cross-sections of an oral product.
Figure 5A illustrates a process diagram for making oral products according to some embodiments.
Figure 5B illustrates an extruder configuration for making oral products according to some embodiments.
Figure 6A illustrates a process diagram for making oral products according to other embodiments.
Figure 6B illustrates an extruder configuration for making oral products according to certain embodiments
Figure 7 illustrates a rod of mouth-stable polymer exiting an extruder die.
Figure 8 illustrates how a cut piece of mouth-stable polymer including fibers and/or additives can pillow.
Figures 9A and 9B depict compression and springiness test results.
Figure 9C depicts some of the samples tested.
Figures 10A and 10B depict cumulative release timelines for menthol and nicotine from oral products.

### DETAILED DESCRIPTION

The oral products described herein include a mouth-stable polymer matrix as defined in the claims and one or more additives. The one or more additives can be dispersed in the mouth-stable polymer matrix such that the one or more additives are released from the oral product when the oral product is received within the oral cavity and exposed to saliva. The oral products described herein can provide a favorable additive release profile and tactile experience.

Suitable mouth-stable polymers are defined in the claims and include polyurethane, which is a thermoplastic elastomer. As used here, the term "mouth stable" means that the polymer does not appreciably dissolve or disintegrate when exposed to saliva within an oral cavity and at the normal human body temperature of about 37°C (98.6 °F)) over a period of one hour. In addition to biostable polymers, mouth-stable polymers can include biodegradable polymers that breakdown over periods of days, weeks, months, and/or years, but do not appreciably break down when held in an oral cavity and exposed to saliva for a period of one hour. In some embodiments, the mouth-stable polymer is stable within an oral cavity and exposed to saliva at the normal human body temperature for a period of at least 6 hours, at least 12 hours, at least 24 hours, or at least 2 days. Accordingly, the oral products described herein can remain intact when placed within an oral cavity during a use period. After use, the mouth-stable polymer matrix can be removed from the oral cavity and discarded.

The mouth-stable polymer can have shape stability. In some cases, the oral product 110 can be chewed without significant and instantaneous permanent plastic deformation. As the oral product 100 is chewed, it can become more pliable and additional additives can become available for release into the oral cavity. Some embodiments of the oral product 110 can be adapted to remain non-sticky during and after use. After prolonged use, certain embodiments of the oral product 110 will expand and become flatter. The oral product, however, can retain the essence of its original shape.

One or more additives are included in the oral product and adapted to be released from the oral product when the oral product is placed in an oral cavity. The oral product, in some embodiments, includes nicotine. The oral product can include a combination of nicotine, sweeteners, and flavorants to mimic the flavor profile and tactile experience of certain tobacco products (e.g., a pouched smokeless tobacco product).

In some embodiments, a nicotine-containing oral product can be substantially free of tobacco plant tissue. As used herein, the term "tobacco plant tissue" refers to processed or non-processed cellulosic parts (e.g., leaves, stems) of a member of the genus *Nicotiana,* but does not include extracts of tobacco (e.g., tobacco-derived nicotine). For example, an oral product can include one or more organoleptic components extracted from raw or processed tobacco, yet be substantially free of tobacco plant tissue.

In addition to additives, sweeteners, and flavorants, the oral product can also include fibers, fillers, plasticizers, and/or processing aids. Fibers can help provide access to the additives, sweeteners, and/or flavorants. As will be discussed below, fibers can provide channels for additives, sweeteners, and/or flavorants to leach out of the mouth-stable polymer matrix. The fiber-polymer matrix can absorb one or more additives and provide a pathway for one or more additives to be released from the oral product. The fiber-polymer matrix can be porous. In some embodiments, the fiber-polymer matrix can have a plurality of pores having a pore diameter of between 40 microns and 60 microns and a plurality of pores having a pore diameter of between 1 micron and 10 microns. During use, saliva can be absorbed into the fiber-polymer matrix to release the additives, sweeteners, and/or flavorants. The absorbed saliva can enter the pores and/or cause the fibers to expand, which can facilitate further release of additives, sweeteners, and/or flavorants. Mechanical action (e.g., chewing) of the oral product can facilitate the release of the additives, sweeteners, and/or flavorants.

Fillers can also be included in the mouth-stable polymer matrix to alter the texture or pliability of the oral product. The mouth-stable polymer matrix can also include plasticizers, which can increase the softness of the oral product. Processing aids can also be present in the oral product and be used to facilitate shaping processes.

### Oral Product Shapes and Packaging

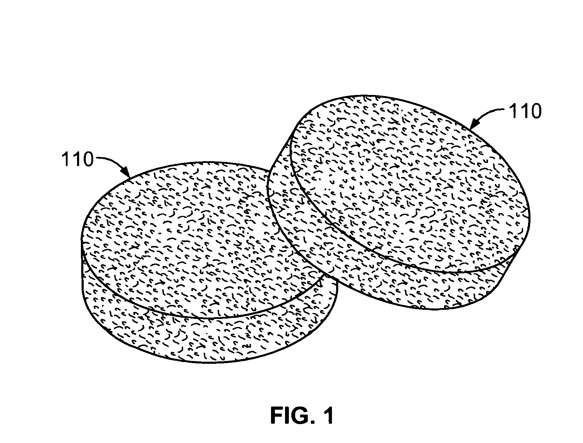

Figure 1 depicts an example of an oral product 110. The oral product 110 has a disk shape. For example, the oral product 110 can have a diameter of about 12 mm and a thickness of about 2.5 mm.

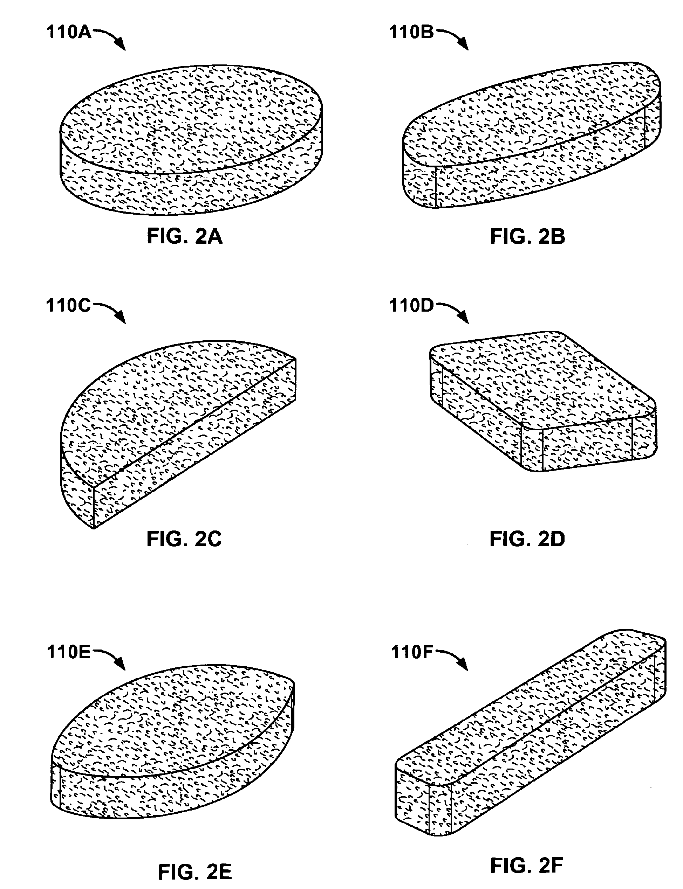

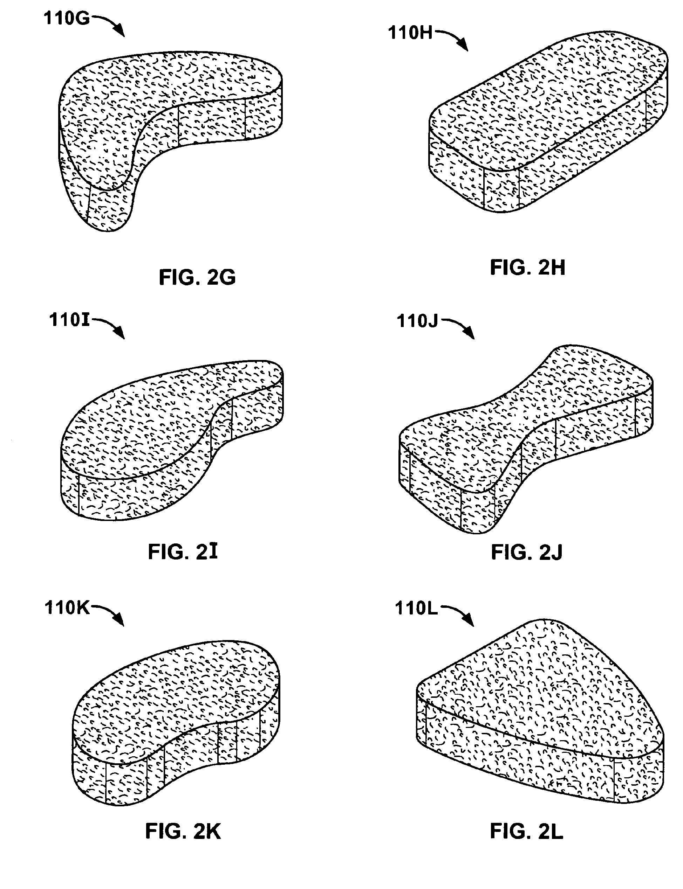

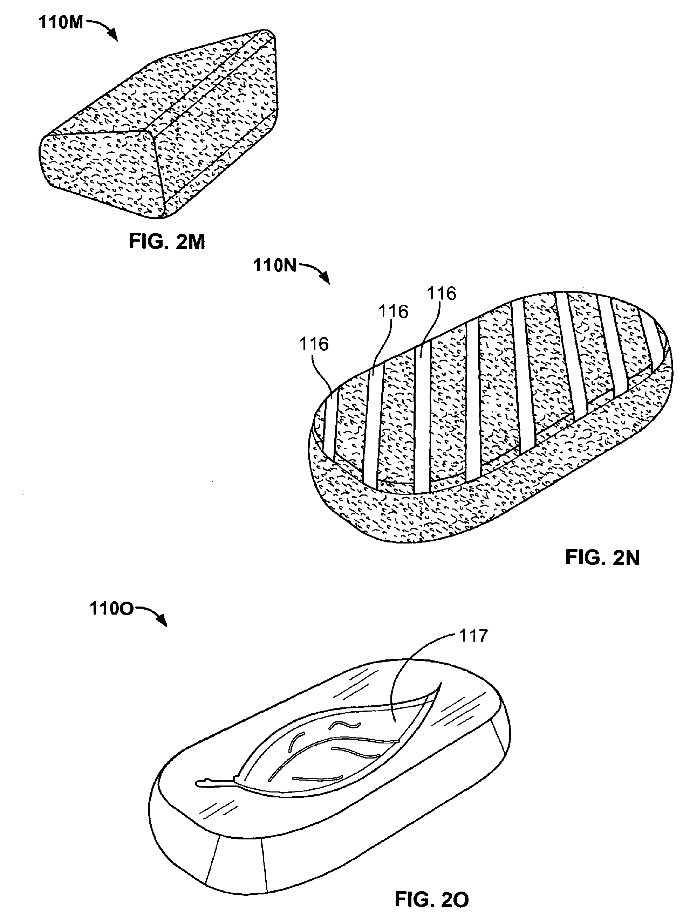

Referring now to FIGS. 2A-2N, the oral product 110 can be molded into any desired shape. For example, referring to Figures 2A-2L, the oral product 110A-L can be formed in a shape that promotes improved oral positioning in the oral cavity, improved packaging characteristics, or both. In some circumstances, the oral product 110A-L can be configured to be: (A) an elliptical-shaped oral product 110A ; (B) an elongated elliptical-shaped oral product 110B; (C) semi-circular oral product 110C; (D) square or rectangular-shaped oral product 110D; (E) football-shaped oral product 110E; (F) elongated rectangular-shaped oral product 110F; (G) boomerang-shaped oral product 110G; (H) rounded-edge rectangular-shaped oral product 110H; (I) teardrop- or comma-shaped oral product 110I; (J) bowtie-shaped oral product 110J; (K) peanut-shaped oral product 110K; and (L) shield-shaped oral product. Alternatively, the oral product can have different thicknesses or dimensionality, such that a beveled article (e.g., a wedge) is produced (see, for example, product 110M depicted in FIG. 2M) or a hemi-spherical shape is produced. In some embodiments, the oral product has a shield shape.

In addition or in the alternative to flavorants being included within the mouth-stable polymer matrix, flavorants can be included on an exterior of the oral product 110. For example, referring to FIG. 2N some embodiments of an oral product 110N can be equipped with flavor strips 116.

Referring to FIG. 2O, particular embodiments of the oral product 110 can be embossed or stamped with a design (e.g., a logo, an image, or the like). For example, the oral product 110O can be embossed or stamped with any type of design 117 including, but not limited to, a trademark, a product name, or any type of image. The design 117 can be formed directly into the oral product, arranged along the exterior of the product 110O. The design 117 can also be embossed or stamped into those embodiments with a dissolvable film 116 applied thereto.

In some embodiments, the oral product 110 or products 110A-O can be wrapped or coated in an edible or dissolvable film, which may be opaque, substantially transparent, or translucent. The dissolvable film can readily dissipate when the oral product 110 is placed in an oral cavity. In some embodiments, the oral product 110 can be coated with a mouth-stable material. Exemplary coating materials include Beeswax, gelatin, acetylated monoglyceride, starch (e.g., native potato starch, high amylose starch, hydroxypropylated potato starch), Zein, Shellac, ethyl cellulose, methylcellulose, hydroxypropyl methylcellulose, carboxymethyl cellulose, and combinations thereof. For example, a coating can include a combination of gelatin and methylcellulose. In some embodiments, a coating material can include a plasticizer. In some case, a coating can include a colorant, a flavorant, and/or a one or more of the additives discussed above. For example, a coating can include nicotine to provide a user with an initial nicotine burst. In some cases, the matrix of mouth-stable polymer 120 can have surfaces roughened to improve the adherence of a coating. In some cases, a coating can provide a glossy or semi-glossy appearance, a smooth surface, and/or an appealing visual aesthetic (e.g., a nice color). In some embodiments, the coating (e.g., a beeswax, Zein, acetylated monoglyceride, and/or hydroxypropylated potato starch coating) can provide soft mouth feel. In some embodiments, the coating (e.g., a methylcellulose, hydroxypropyl methylcellulose, carboxymethyl cellulose, ethyl cellulose, and/or gelatin coating) can provide a hard outer coating.

One or more oral products 110 can be packaged in a variety of conventional and nonconventional manners. For example, a plurality of oral products 110 can be packaged in a container having a lid. In other embodiments, a plurality of oral products 110 can be stacked and packaged in a paper, plastic, and/or aluminum foil tube. The packaging can have a child-resistant lid.

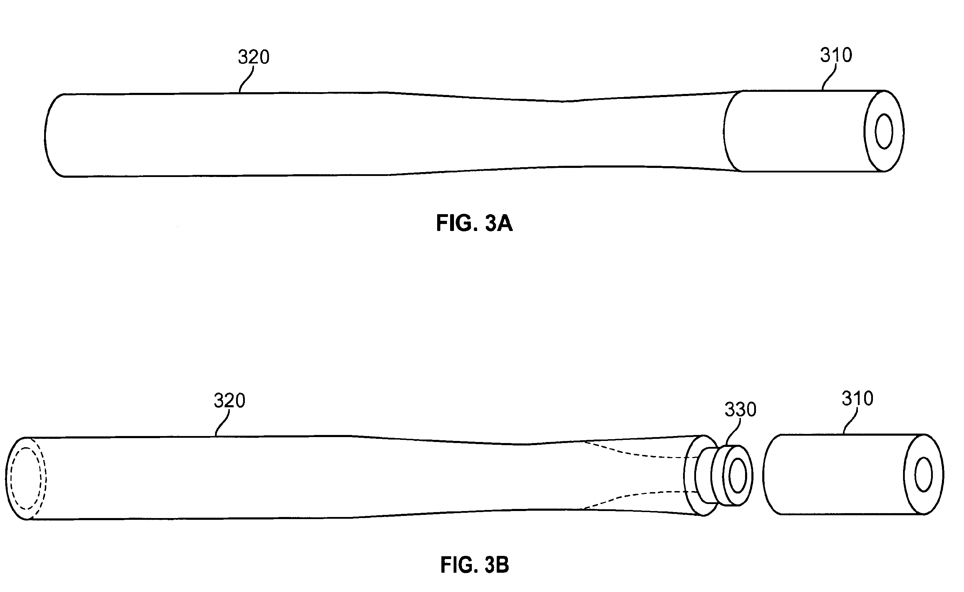

The oral product 110 can also include additional elements. In some embodiments, a mouth-stable polymer matrix including nicotine or a derivative thereof can be attached to a rod, tube, or stick. For example, Figures 3A-3J illustrate tubes attached to a mouth-stable polymer matrix tips. Figure 3A depicts an embodiment of an oral product having a tip piece 310 and a tube piece 320. The tip piece 310 can include the mouth-stable polymer matrix having fibers and/or one or more additives within the polymer matrix. The tip piece 310 can be sized and shaped to be at least partially received in an oral cavity. The tube piece 320 can be made of any conventional polymer. During use the tube piece 320 can act as holder for the tip piece 310. The tube piece 320 and the tip piece 310 can be attached by a snap-fit attachment feature 330, as shown in Figure 3B.

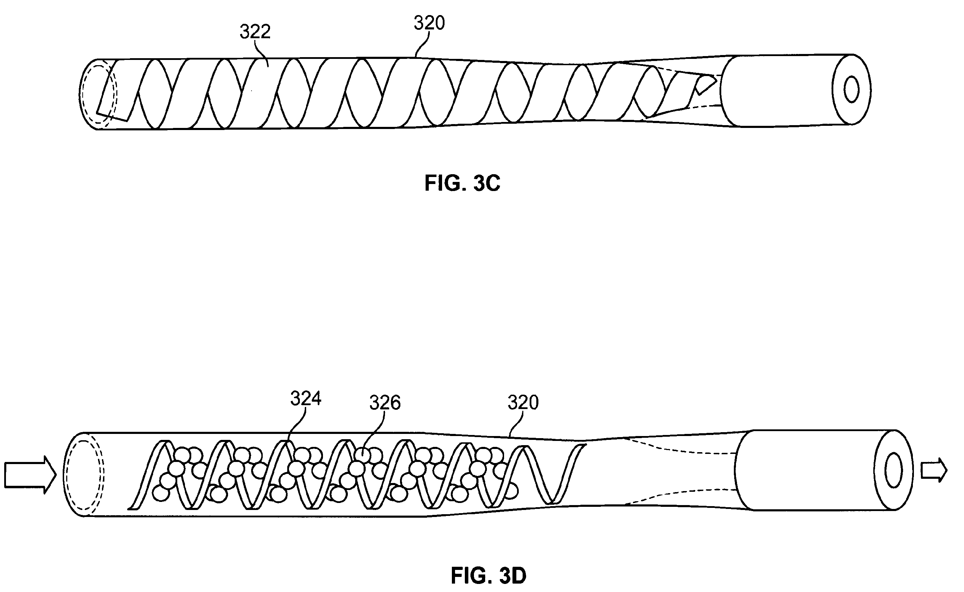

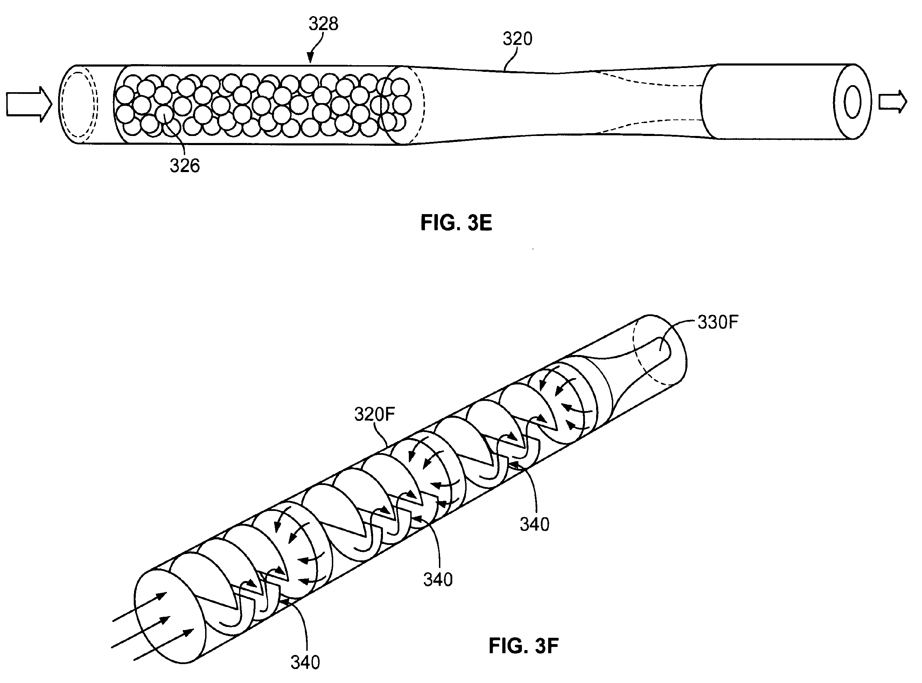

The tube piece 320 can be reusable. For example, multiple tip pieces 310 can be packaged with a single tube piece 320 and a user can switch off the tip pieces 310. In other embodiments, the tube pieces 320 can be intended for a single use. In some embodiments, the tube pieces 320 can include flavorants within the tube. The flavorants can be adapted to be released when air is drawn through the tube 320. For example, Figure 3C depicts a tube including a flavor ribbon 322. Figure 3D depicts a tube 320 including a flavor strip 324 and a plurality of flavor beads 326. Figure 3E depicts a tube 320 including a compressed mass 328 of flavor beads 326. In some embodiments, the inside of the tube can have structure adapted to alter the flow pattern of air drawn into the tube. For example, Figure 3F depicts a tube 320F having a series of steps and constrictions 340 adapted to alter the flow pattern of air drawn into the tube. Figure 3F also depicts an alternative connection feature 330F.

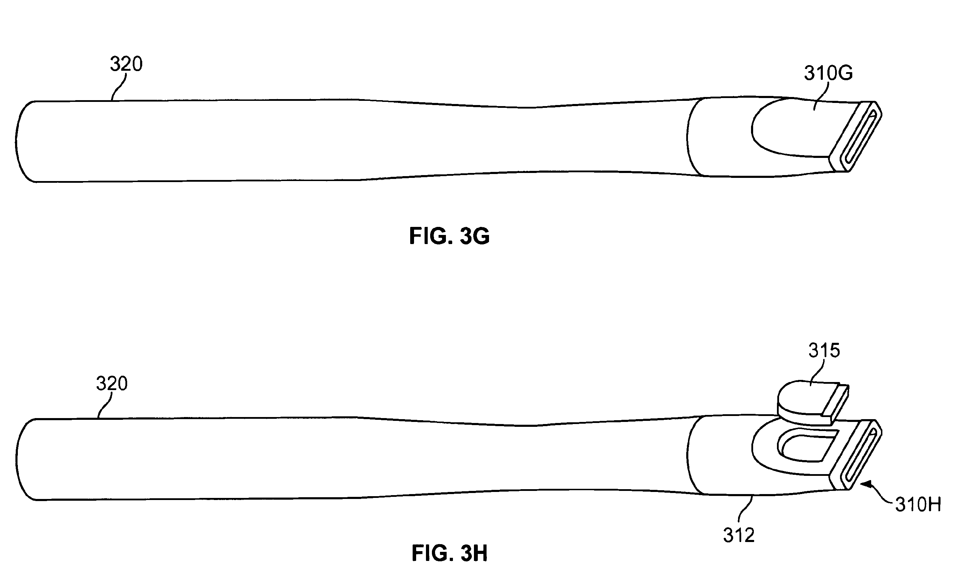

Figure 3G depicts an embodiment having a recorder-like shape. As shown, a tip piece 310G is connected to the contoured tube piece 320. For example, the recorder-shaped tip 310G can be composed of a mouth-stable polymer matrix that includes cellulosic fibers, nicotine, one or more sweeteners, and one or more flavorants. As shown, the tip piece 310G is sized and shaped to be at least partially received within an adult's oral cavity.

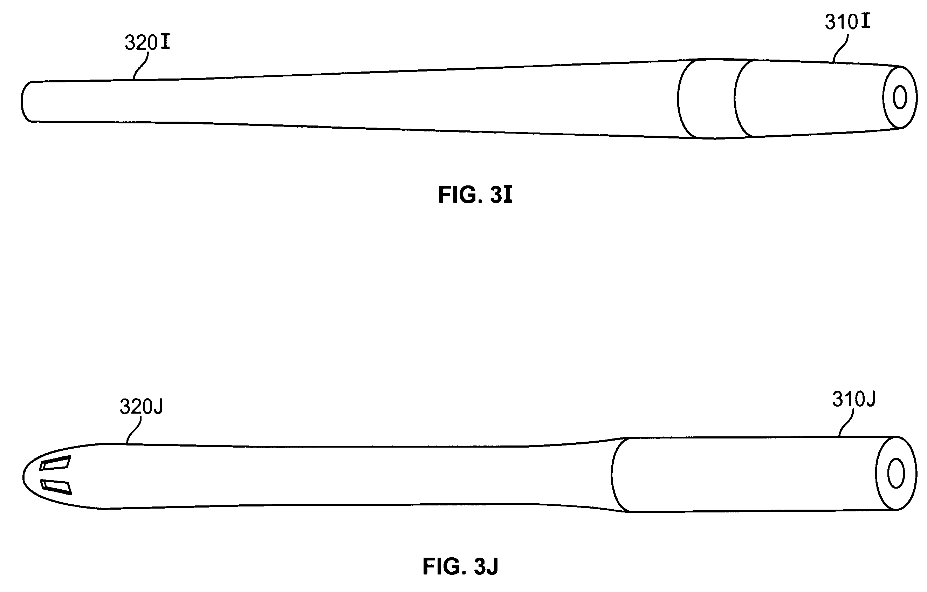

Figure 3H depicts a similarly shaped oral product having a plastic recorder-shaped tip 310H that includes a reusable plastic part 312 and a mouth-stable polymer matrix part 315. Figures 3I and 3J depict embodiments having alternatively shaped tip pieces 310I and 310J. Figure 3I depicts an embodiment having a tapered tube 320I. Figure 3J depicts an embodiment having vent holes at the non-tip end of the tube piece 320J.

In some embodiments, a system or kit of different tubes and rods and/or different tips can be packaged together, each having the same type of attachment features. Embodiments having each of the combinations of tips and tubes or rods shown in FIGS. 3A-3J are contemplated.

### Oral Product Properties

The oral product 110 can provide a favorable tactile experience (e.g., mouth feel). The oral product 110 can also retain its shape during processing, shipping, handling, and optionally use. As noted above, the oral product 110 includes a mouth-stable polymer matrix that does not appreciably dissolve or disintegrate when placed in an oral cavity and exposed to saliva. In some embodiments, the oral product 110 can have an elasticity allowing an adult consumer to work the product within the mouth. In some embodiments, the oral product 110 has at least some shape memory and thus can return to shape after being squeezed between teeth in an oral cavity. Working of the oral product 110 within the oral cavity can accelerate the release of the additives, sweeteners, and/or flavorants within the mouth-stable polymer matrix.

During use, the oral product 110 can absorb saliva into the polymer-fiber matrix. The saliva can cause the polymer-fiber matrix to swell, which can further increase access to different sections of the polymer-fiber matrix. Physical activity, such as chewing of the product in the mouth, can also accelerate the polymer-matrix swelling and therefore the release of additives. As the product is chewed, saliva can access different sections of the polymer-fiber matrix. The mouth-stable polymer can have shape stability. In some cases, the oral product 110 can be chewed without significant and instantaneous permanent plastic deformation (such as that experienced by a chewing gum when chewed). As the oral product 100 is chewed, it can become more pliable and additional additives can become available for release into the oral cavity. Some embodiments of the oral product 110 can be adapted to remain non-sticky during and after use. After prolonged use, certain embodiments of the oral product 110 will expand and become flatter. The oral product, however, can retain the essence of its original shape. The amount of deformation will depend on the duration of use and an amount of mouth force used. As the product is used, it can increase in both weight and volume, due to the swelling. Simulated chewing tests using artificial saliva show a weight increases ranging from 4% to 75% depending on the selection of experimental parameters. With greater the physical manipulation, the oral product 110 will have a greater amount of swelling and thus have a larger weight gain. In certain embodiments, the oral product 110 will have an increase in weight of between 4 and 75 percent when chewed by an adult consumer for 30 minutes.

One way of characterizing the properties of the oral product is by measuring the compressibility and springiness of the product. The compressibility can be calculated as a percentage of reduction in thickness of the sample when the sample is compressed with a standardized probe with a particular force. As used herein, the term "compression @ 250 N test" defines a test of a sample where the sample is placed on a flat stationary surface and twice compressed with a 10mm-diameter-sphere-tipped probe with a force of 250 N with a hold time of 30 seconds between compressions. The "percentage of compression @ 250 N" is the maximum amount of reduction in thickness of the sample during the compression @250 N test. For example, if a 3mm thick sample is compressed to a minimum thickness of 1.5 mm during either of the two compressions, the sample is said to have a 50% compression @ 250 N. As used herein, the term "compression @ 425 N test" defines a test of a sample where the sample is placed on a flat stationary surface and twice compressed with a 10mm-diameter-sphere-tipped probe with a force of 425 N with a hold time of 30 seconds between compressions. For comparison, a normal human bite force is typically between 400 and 500 N.

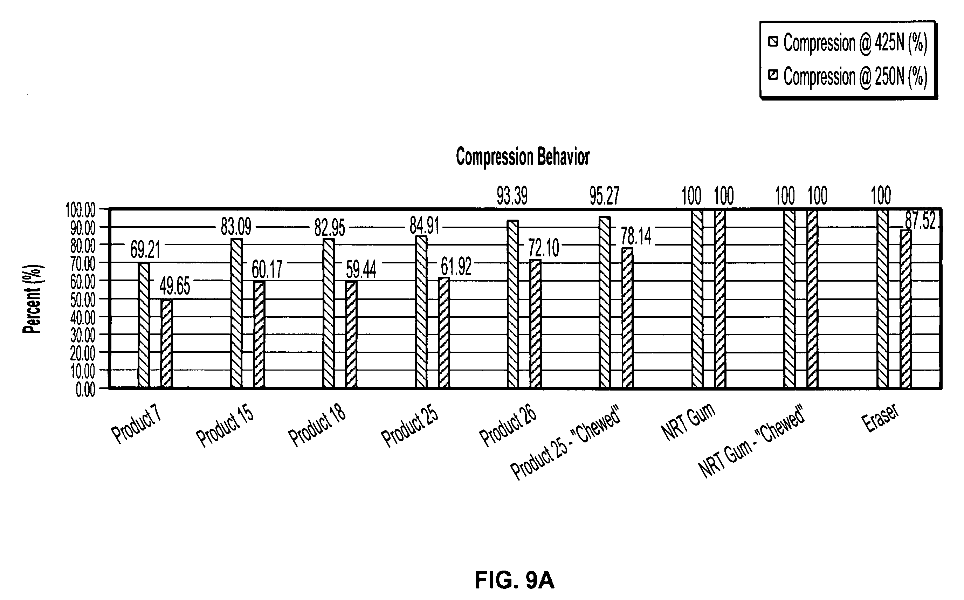

In some embodiments, the oral product 110 has a percentage of compression @ 250 N of less than 95%. In certain embodiments, the oral product 110 has a percentage of compression @ 250 N of less than 90%, less than 85%, or less than 80%. In certain embodiments, the oral product 110 has a percentage of compression @ 250 N of at least 10%, at least 25%, or at least 40%. For example, the oral product can have a percentage of compression @ 250 N of between 45% and 80%. In some embodiments, the oral product 110 has a percentage of compression @ 425 N of less than 99%. In certain embodiments, the oral product 110 has a percentage of compression @ 425 N of less than 98%, less than 97%, or less than 96%. In certain embodiments, the oral product 110 has a percentage of compression @ 425 N of at least 10%, at least 25%, at least 50%, or at least 60%. For example, the oral product can have a percentage of compression @ 425 N of between 65% and 98%. FIG. 9A, discussed in more detail below, depicts examples of compression test results for certain embodiments of oral products, for gum (both chewed and fresh), and for an eraser.

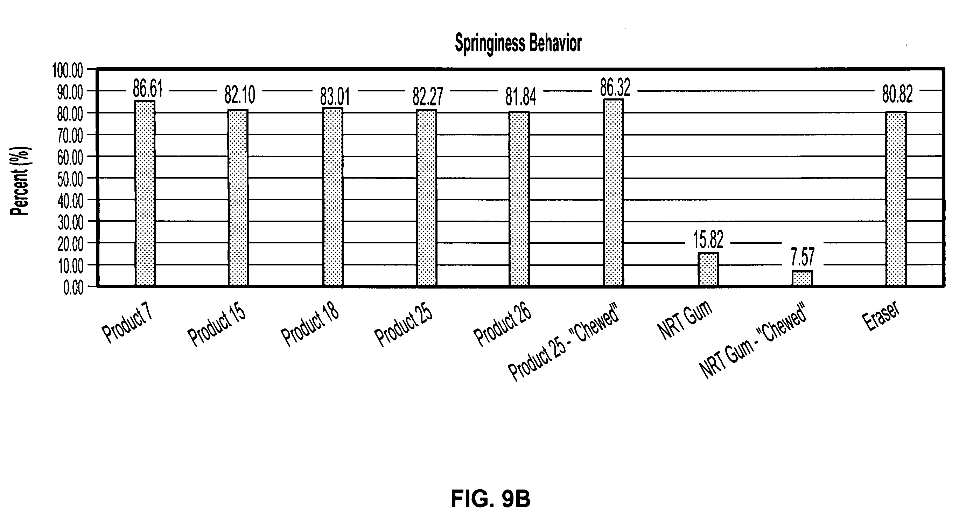

The springiness of a sample can be measured by measuring the percenage of recovery after a sample is compressed. As used herein, the term "percentage of springiness" means the percentage of thickness recovery of the sample during a 30 second recovery time after being compressed by the compression @ 425 N test using the 10mm-diameter-sphere-tipped probe. For example, if a sample is compressed from an original thickness of 3.0mm to a thickness of 2.0mm and then recovers to 2.5mm after 30 seconds, the springiness of the sample would be 50%. In some embodiments, the oral product 110 has a percentage of springiness of at least 20%. In certain embodiments, the oral product 110 has a percentage of springiness of at least 40%, at least 50%, at least 60%, at least 70%, at least 75%, or at least 80%. In certain embodiments, the percentage of springiness is less than 95%, less than 90%, or less than 87%. For example, the oral product can have a percentage of springiness of between 75% and 90%. FIG. 9B, discussed in more detail below, depicts examples of springiness test results for certain embodiments of oral products, for gum (both chewed and fresh), and for an eraser.

The particular materials used in the oral product 110 and the processing techniques discussed below can have an impact on the compressibility and springiness of the oral product. In addition to different materials have different compressibility and springiness properties, the incorporation of air bubbles or channels, or different fillers and/or fibers can also have an impact on the elasticity and pliability of the oral product. Additionally, the material properties of the overall oral product 110 can change as additives are released. In some embodiments, fibers and/or fillers can also dissolve or disintegrate during use and thus alter the material properties of the oral product 110 during use.

The oral product 110 can have a variety of colors. In some embodiments, the oral product 110 has an off-white color. In other embodiments, natural and artificial coloring can be added to the mouth-stable polymer before or during the molding process to form oral products 110 having a predetermined color. Encapsulated flavors can be added during the extrusion process to create speckles, patterns or dots within the oral product.

### Polymers

The mouth-stable polymer can be a variety of different biocompatible and biostable polymers. In some embodiments, the mouth-stable polymer is a polymer generally recognized as safe by an appropriate regulatory agency. In some embodiments, the polymer is a thermoplastic polymer. The polymer can also be a thermoplastic elastomer. Mouth-stable polymers according to the invention include polyurethanes, silicone polymers, polyesters, polyacrylates, polyethylenes, polypropylenes, polyetheramides, polystyrenes (e.g., acrylonitrile butadiene styrene, high impact polystyrenes (HIPS)) polyvinyl alcohols, polyvinyl acetates, polyvinyl chlorides, polybutyl acetates, butyl rubbers (e.g., polyisobutylenes), SEBS, SBS, SIS, and mixtures, combinations and copolymers thereof as defined in the claims. In certain embodiments, the mouth-stable polymer is food-grade or medical-grade polymers (e.g., medical-grade polyurethane).

The mouth-stable polymer forms the mouth-stable polymer matrix of the oral product 110. In some embodiments, the oral product includes at least 10 weight percent of one or more mouth-stable polymers. In certain embodiments, the oral product includes at least 20 weight percent, at least 30 weight percent, at least 40 weight percent, at least 50 weight percent, at least 60 weight percent, at least 70 weight percent, at least 80 weight percent, or at least 90 weight percent of one or more mouth-stable polymers. In certain embodiments, the oral product includes between 10 and 90 weight percent of one or more mouth-stable polymers. Accordingly to some embodiments, the oral product includes between 40 and 80 weight percent of the mouth-stable polymers. Some embodiments of the oral product have between 55 and 70 weight percent polymers.

The mouth-stable polymer according to certain embodiments has a flexural modulus of at least 5 MPa when tested according to ASTM Testing Method D790 or ISO 178 at 23 degrees Celsius. In some embodiments, the flexural modulus is at least 10 MPa. For example, the flexural modulus can be between 10 MPa and 30 MPa. In some embodiments, the mouth-stable polymer is a grade that complies with food-contact regulations applicable in one or more countries (e.g., US FDA regulations). In some embodiments, the mouth-stable polymer can be a polyurethane, SIS, or other thermal plastic elastomer meeting the requirements of the FDA-modified ISO 10993, Part 1 "Biological Evaluation of Medical Devices" tests with human tissue contact time of 30 days or less. The mouth-stable polymer can have a shore Hardness of 50D or softer, a melt flow index of 3g/10 min at 200°C/10kg, a tensile strength of 10 MPa or more (using ISO 37), and a ultimate elongation of less than 100% (using ISO 37).

### Additives

A variety of additives can be included in the oral product 110. The additives can include alkaloids (e.g., nicotine), minerals, vitamins, dietary supplements, nutraceuticals, energizing agents, soothing agents, coloring agents, amino acids, chemsthetic agent, antioxidants, food grade emulsifiers, pH modifiers, botanicals (e.g., green tea), teeth whitening (e.g., SHRIMP), therapeutic agents, sweeteners, flavorants, and combinations thereof. In certain embodiments, the additives include nicotine, sweeteners, and flavorants. With certain combinations of nicotine, sweeteners, and flavorants, the oral product may provide a flavor profile and tactile experience similar to certain tobacco products.

### Nicotine

Nicotine within the oral product can be tobacco-derived nicotine, synthetic nicotine, or a combination thereof. In certain embodiments, the oral product includes between 0.1 mg and 6.0 mg of nicotine. In some of these embodiments, the oral product includes between 1.0 mg and 3.0 mg of nicotine.

Tobacco-derived nicotine can include one or more other tobacco organoleptic components other than nicotine. The tobacco-derived nicotine can be extracted from raw (e.g., green leaf) tobacco and/or processed tobacco. Processed tobaccos can include fermented and unfermented tobaccos, dark air-cured, dark fire cured, burley, flue cured, and cigar filler or wrapper, as well as the products from the whole leaf stemming operation. The tobacco can also be conditioned by heating, sweating and/or pasteurizing steps as described in U.S. Publication Nos. 2004/0118422 or 2005/0178398. Fermenting typically is characterized by high initial moisture content, heat generation, and a 10 to 20% loss of dry weight. *See, e.g.,* U.S. Patent Nos. 4,528,993; 4,660,577; 4,848,373; and 5,372,149. By processing the tobacco prior to extracting nicotine and other organoleptic components, the tobacco-derived nicotine may include ingredients that provide a favorable experience.

The tobacco-derived nicotine can be obtained by mixing cured and fermented tobacco with water or another solvent (e.g., ethanol) followed by removing the insoluble tobacco material. The tobacco extract may be further concentrated or purified. In some embodiments, select tobacco constituents can be removed. Nicotine can also be extracted from tobacco in the methods described in the following patents: U.S. Patent Nos. 2,162,738; 3,139,436; 3,396,735; 4,153,063; 4,448,208; and 5,487,792.

The nicotine can also be purchased from commercial sources, whether tobacco-derived or synthetic. In other embodiments, the oral product can include a derivative of nicotine (e.g., a salt of nicotine).

### Antioxidants

The oral product 110 can also include one or more antioxidants. In some embodiments, an oral product 110 can include a combination of nicotine and antioxidants. Antioxidants can result in a significant reduction in the conversion of nicotine into nicotine-N-oxide when compared to oral products without antioxidants. In some cases, an oral product can include 0.01 and 5.00 weight percent antioxidant, between 0.05 and 1.0 weight percent antioxidant, between 0.10 and 0.75 weigh percent antioxidant, or between 0.15 and 0.5 weight percent antioxidant. Suitable examples of antioxidants include ascorbyl palmitate (a vitamin C ester), BHT, ascorbic acid (Vitamin C), and sodium ascorbate (Vitamin C salt). In some embodiments, monosterol citrate, tocopherols, propyl gallate, tertiary butylhydroquinone (TBHQ), butylated hydroxyanisole (BHA), Vitamin E, or a derivative thereof can be used as the antioxidant. For example, ascorbyl palmitate can be the antioxidant in the formulations listed in Table I. Antioxidants can be incorporated into the polymer (e.g., polyurethane) during an extrusion process or after the polymer is extruded (e.g., during a post-extrusion flavoring process).

Table I (below) compares a test sample with a control sample to compare how the inclusion of antioxidant in an oral product 110 impacts the about of nicotine-N-oxide formed in the oral product after aging for 2 weeks and 4 weeks. The ambient samples were held at 25 °C and 65% relative humidity. Both the control and test samples included 1.5 mg of nicotine per piece. As shown, the inclusion of antioxidant significantly reduces the amount of nicotine-N-oxide formed in the oral product 110.

**Table I**

| | Nicotine N Oxide | | % Difference between Test and Control |
|---|---|---|---|
| | Control* | Test** | |
| Week 0 | 0.023% | 0.035% | -- |
| Ambient Week 2 | 0.510% | 0.156% | -69.49% |
| Ambient Week 4 | 0.735% | 0.261% | -64.51% |
| Ambient Week 6 | 0.893% | 0.277% | -69.04% |
| Ambient Week 8 | 0.950% | 0.449% | -52.68% |
| Ambient Week 10 | 0.890% | 0.491% | -44.87% |
| Ambient Week 12 | 1.009% | 0.539% | -46.64% |

| | | | |
|---|---|---|---|
| * The control is free of antioxidant, but includes 1.5 mg nicotine. ** The test includes about 0.15% ascorbyl palmitate antioxidant and about 1.5 mg nicotine. | | | |

In some cases, the oral product 110 can have a conversion of less than 0.50 % of nicotine into nicotine-N-oxide after aging the oral product 110 for 2 weeks at 25 °C and 65% relative humidity. In some cases, the oral product 110 can have a conversion of less than 0.20% of nicotine into nicotine-N-oxide after aging the oral product 110 for 2 weeks at 25 °C and 65% relative humidity. In some cases, the oral product 110 can have a conversion of less than 0.70% of nicotine into nicotine-N-oxide after aging the oral product 110 for 4 weeks at 25 °C and 65% relative humidity. In some cases, the oral product 110 can have a conversion of less than 0.30% of nicotine into nicotine-N-oxide after aging the oral product 110 for 4 weeks at 25 °C and 65% relative humidity. In some cases, the oral product 110 can have a conversion of less than 0.80 % of nicotine into nicotine-N-oxide after aging the oral product 110 for 6 weeks at 25 °C and 65% relative humidity. In some cases, the oral product 110 can have a conversion of less than 0.40% of nicotine into nicotine-N-oxide after aging the oral product 110 for 6 weeks at 25 °C and 65% relative humidity. In some cases, the oral product 110 can have a conversion of less than 0.30% of nicotine into nicotine-N-oxide after aging the oral product 110 for 6 weeks at 25 °C and 65% relative humidity. In some cases, the oral product 110 can have a conversion of less than 0.85 % of nicotine into nicotine-N-oxide after aging the oral product 110 for 8 weeks at 25 °C and 65% relative humidity. In some cases, the oral product 110 can have a conversion of less than 0.50% of nicotine into nicotine-N-oxide after aging the oral product 110 for 8 weeks at 25 °C and 65% relative humidity. In some cases, the oral product 110 can have a conversion of less than 0.85 % of nicotine into nicotine-N-oxide after aging the oral product 110 for 10 weeks at 25 °C and 65% relative humidity. In some cases, the oral product 110 can have a conversion of less than 0.55% of nicotine into nicotine-N-oxide after aging the oral product 110 for 10 weeks at 25 °C and 65% relative humidity. In some cases, the oral product 110 can have a conversion of less than 0.95 % of nicotine into nicotine-N-oxide after aging the oral product 110 for 12 weeks at 25 °C and 65% relative humidity. In some cases, the oral product 110 can have a conversion of less than 0.60% of nicotine into nicotine-N-oxide after aging the oral product 110 for 12 weeks at 25 °C and 65% relative humidity.

The presence of antioxidant can also reduce the formation of other tobacco derived impurities, such as Cotinine and myosime, as shown in Table II (below). Antioxidant level 1 is 0.075% in the formula and level 2 is 0.15% in the formula. The control sample includes no antioxidant. The antioxidant is ascorbyl palmitate.

**Table II**

| *Week 4* | | | | | | | | | |
|---|---|---|---|---|---|---|---|---|---|
| Storage Conditions | Packaging Type | % Relative to Nicotine Concentration (Average, n=3) | | | | | | | |
| | | Myosmine | Nornicotine | Anabasine | Cotinine | Anatabine | Nicotine-Oxide | b-Nicotydine | Total |
| Ambient (25°C,60 RH) | Control | 0.125% | 0.014% | ND | 0.023% | ND | 0.735% | 0.017% | 0.913% |
| | AD Level 1 | 0.041% | 0.018% | ND | 0.007% | ND | 0.188% | 0.009% | 0.263% |
| | A0 Level 2 | 0.038% | 0.029% | ND | 0007% | ND | 0.261% | 0.008% | 0.343% |

### Sweeteners

A variety of synthetic and/or natural sweeteners can be used as additives in the oral product 110. Suitable natural sweeteners include sugars, for example, monosaccharides, disaccharides, and/or polysaccharide sugars, and/or mixtures of two or more sugars. According to some embodiments, the oral product 110 includes one or more of the following: sucrose or table sugar; honey or a mixture of low molecular weight sugars not including sucrose; glucose or grape sugar or corn sugar or dextrose; molasses; corn sweetener; corn syrup or glucose syrup; fructose or fruit sugar; lactose or milk sugar; maltose or malt sugar or maltobiose; sorghum syrup; mannitol or manna sugar; sorbitol or d-sorbite or d-sobitol; fruit juice concentrate; and/or mixtures or blends of one or more of these ingredients. The oral product 110 can also include non-nutritive sweeteners. Suitable non-nutritive sweeteners include: stevia, saccharin; Aspartame; sucralose; or acesulfame potassium.

### Flavorants

The oral product 110 can optionally include one or more flavorants. The flavorants can be natural or artificial. For example, suitable flavorants include wintergreen, cherry and berry type flavorants, various liqueurs and liquors (such as Dramboui, bourbon, scotch, and whiskey) spearmint, peppermint, lavender, cinnamon, cardamon, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, anise, coriander, coffee, liquorish, and mint oils from a species of the genus Mentha, and encapsulated flavors. Mint oils useful in particular embodiments of the oral product 110 include spearmint and peppermint. Synthetic flavorants can also be used. In certain embodiments, a combination of flavorants can be combined to imitate a tobacco flavor. The particular combination of flavorants can be selected from the flavorants that are generally recognized as safe ("GRAS") in a particular country, such as the United States. Flavorants can also be included in the oral product as encapsulated flavorants.

In some embodiments, the flavorants in the oral product 110 are limited to less than 20 weight percent in sum. In some embodiments, the flavorants in the oral product 110 are limited to be less than 10 weight percent in sum. For example, certain flavorants can be included in the oral product 110 in amounts of about 1 weight percent to 5 weight percent.

### Other Additives

The oral product 110 may optionally include other additives. For example, these additives can include non-nicotine alkaloids, dietary minerals, vitamins, dietary supplements, therapeutic agents, and fillers.

Oral products 110 can also include vitamins, dietary minerals, other dietary supplements, and/or therapeutic agents. For example, suitable vitamins include vitamins A, B1, B2, B6, C, D2, D3, E, F, K, and P. For example, an oral product 110 can include C-vitamins with nicotine. Suitable dietary minerals include calcium (as carbonate, citrate, etc.) or magnesium (as oxide, etc.), chromium (usually as picolinate), and iron (as bis-glycinate). One or more dietary minerals could be included in an oral product with or without the use of other additives. Other dietary supplements and/or therapeutic agents can also be included as additives.

In some embodiments, the oral product 110 includes a therapeutic agent that is preferable absorbed transbuccally. For example, so therapeutic agents do not pass into the blood stream if they are swallowed. Exemplary therapeutic agents that can be included in an oral product 110 provided herein can include Gerd, Buprenorphin, Nitroglycerin, Diclofenac, Fentanyl, Carbamazepine, Galantamine, Acyclovir, Polyamidoamine Nanoparticles, Chlorpheniramine, Testosterone, Estradiol, Progesterone, Calcitonin, Fluorouracil, Naltrexone, Odansetron, Decitabine, Selegiline, Lamotrigine, and Prochlorperazine. For example, an oral product 110 can include Buprenorphine and be used for pain treatment. In some embodiments, an oral product 110 can include Nitroglycerin and be used for Angina Pectoris treatment. Because of the release properties of the oral product 110, therapeutic agents included therein can be released at a rate such that a majority of the therapeutic agent is absorbed transbuccally, rather than swallowed.

The oral product 110 can also include fillers such as starch, di-calcium phosphate, lactose, sorbitol, mannitol, and microcrystalline cellulose, calcium carbonate, dicalcium phosphate, calcium sulfate, clays, silica, glass particles, sodium lauryl sulfate (SLS), glyceryl palmitostearate, sodium benzoate, sodium stearyl fumarate, talc, and stearates (e.g., Mg or K), and waxes (e.g., glycerol monostearate, propylene glycol monostearate, and acetylated monoglycerides), stabilizers (e.g., ascorbic acid and monosterol citrate, BHT, or BHA), disintegrating agents (e.g., starch, sodium starch glycolate, cross caramellose, cross linked PVP), pH stabilizers, or preservatives. In some embodiments, the amount of filler in the oral product 110 is limited to less than 10 weight percent in sum. In some embodiments, the amount of filler in the oral product 110 is limited to be less than 5 weight percent in sum. In some embodiments, the fillers are mouth stable. In other embodiments, the fillers can dissolve or disintegrate during use and thus result in an oral product that becomes more pliable during use.

### Fibers

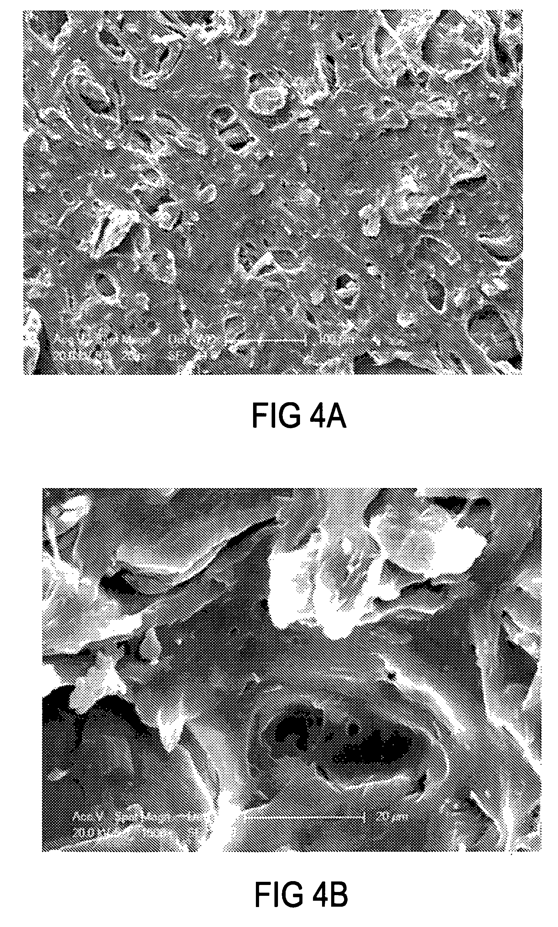

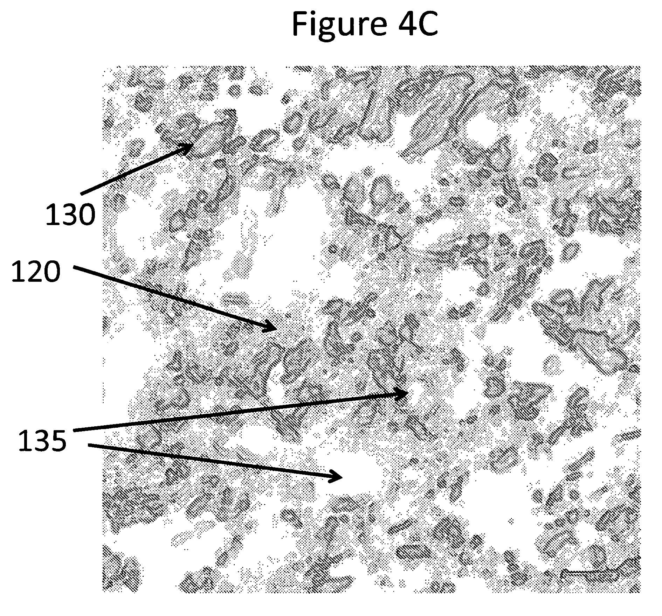

The oral product can include fibers within the mouth-stable polymer matrix. As will be discussed below, the fibers can be mixed with the mouth-stable polymer prior to or during an extrusion process. As shown in Figure 4, the fibers provide passages in the mouth-stable polymer matrix, which can permit certain additives within the mouth-stable polymer matrix to be released into an oral c: vity when the oral product is received in an oral cavity and exposed to saliva. The additives can be absorbed in fiber-polymer matrix and/or form pockets within the mouth-stable polymer matrix, which can be accessed via the.fibers 130.

Figures 4A-4C depicts cross-sections of oral products 100. Figures 4A and 4B are images from a scanning electron microscope (SEM) (XL30 ESEM TMP, FEI/Philips, the Netherlands). The SEM microscope was operated at 20kV electron acceleration voltage and images were recorded at different magnifications. Figure 4A is an SEM image showing the porous structure of the mouth stable polymer matrix 120. Figure 4A highlights pores 135a having diameters of between 20 and 60 microns. Figure 4B is also an SEM image showing the porous structure of the mouth stable polymer matrix 120. Figure 2B, however, has an increased magnification, thus pores 135b having diameters of between 1 and 10 microns can be seen.

Figure 4C is a confocal laser scanning microscopy (CLSM) image. In CLSM, only fluorescent labeled materials are visible. The product was stained with the fluorescent dyes of acridinorange and acriflavine, respectively. The light source was an Argon laser using λex=488 nm (acridinorange) and a HeNE laser using λex=594 nm (acriflavin). The signals were emitted in the wavelength interval of 500-550 nm and 610-650 nm. Figure 4C was converted into gray scale for the purposes of this application. The darker areas show the placement of fibers 130 in the mouth-stable polymer matrix. The lighter grey areas show the mouth-stable polymer 120 (in this case, polyurethane). The white areas in the image are pores in the structure.. In some embodiments, the fibers are hydrophilic such that water-soluble additives can be wicked by the fibers. In some embodiments, the fibers can dissolve to leave channels. Additives can be present in the pores 135 of the mouth-stable polymer matrix 120.

The fibers can be cellulosic fibers. The cellulosic fibers can be derived from plant tissue. In some embodiments, the cellulosic fibers include cellulose. The cellulosic fibers can further include lignin and/or lipids. Suitable sources for cellulosic fibers include wood pulp, cotton, sugar beets, bran, citrus pulp fiber, switch grass and other grasses, Salix (willow), tea, and Populus (poplar). In some embodiments, the cellulosic fibers can be chopped or shredded plant tissue comprising various natural flavors, sweeteners, or active ingredients. In some embodiments, the oral product 110 can include nicotine as an additive (optionally with additional sweeteners and flavors) and non-tobacco cellulosic fiber, and thus be substantially free of tobacco plant tissue.

The cellulosic fibers can have a variety of dimensions. The dimensions of the fibers (in addition to the amount) can impact the release characteristics of the additives. For example, cellulosic fibers can be hydrophilic, thus water soluble additives (e.g., nicotine) can preferentially be absorbed in fiber-polymer matrix. As will be discussed in the Examples below, the release profile of nicotine from a polyurethane oral product 110 can be impacted by both the fiber sizes and the amounts of fiber. In certain embodiments, the cellulosic fiber can be processed to have an average fiber size of less than 200 micrometers. In particular embodiments, the fibers are between 75 and 125 micrometers. In other embodiments, the fibers are processed to have a size of 75 micrometers or less.

The oral product 110 can also include soluble fibers. The soluble fibers can be adapted to dissolve when exposed to saliva when the oral product 110 is received in an oral cavity. In some embodiments, the soluble fiber can be a maltodextrin. The maltodextrin can be derived from com. For example, Soluble Dietary Fiber can be included in an oral product 110. Soluble fibers can be used alone or with cellulosic fibers to provide channels 135 for additives 140 and/or 142 to be released from the oral product 110. As the soluble fibers dissolve, the oral product 110 can become more flexible and the additional channels can open up to permit the release of additional additive deposits 140 or 142. Suitable soluble fibers include psyllium fibers. In other embodiments, the fibers can be partially soluble. For example, sugar beet fibers can partially dissolve during use.

In some embodiments, an oral product 110 can include a combination of soluble and insoluble fibers. The ratio of soluble to insoluble fiber can impact the softness of texture of the oral product 110. The ratio of soluble to insoluble fiber can also impact the compressibility of the oral product 110. In some embodiments, a ratio of soluble to insoluble fiber is between 1:60 and 60:1. In some embodiments, the ratio of soluble to insoluble fiber is greater than 1:50, greater than 1:40, greater than 1:30, greater than 1:20, greater than 1:10, or greater than 1:5. In some embodiments, the ratio of soluble to insoluble fiber is less than 1:1, less than 1:2, less than 1:5, less than 1:10, less than 1:20, or less that 1:30. In some case, an oral product having a mixture of soluble and insoluble fibers can have a percentage of compression @ 250 N of between 60 percent and 98 percent, between 65 percent and 95 percent, between 70 percent and 90 percent, or between 80 and 89 percent.

Table III, below, depicts the percentage of compression @ 250 N for oral products having different percentages of soluble and insoluble fibers. As shown, the inclusion of soluble fiber can increase the compressibility of the oral product, which can also be perceived as a softer mouth feel by an adult tobacco consumer. The soluble and the insoluble fiber depicted in Table I were pre-mixed and added into the process via a single feeder, but separate fiber feeders can also be added. For example, larger commercial processes can include additional fiber feeders to meter the insoluble and soluble fiber separately to produce a desired ratio.

**Table III.**

| Prototype # | Compression @250N | Soluble Fiber FiberSol-2™ | Insoluble Fiber | Polyurethane | Antioxidant |
|---|---|---|---|---|---|
| Control | 60% | 0.0% | 31.392% | 56.505% | 0.0% |
| Sample A | 72% | 1.162% | 30.981% | 55.765% | 0.148% |
| Sample B | 83% | 2.351% | 28.994% | 56.421% | 0.15% |
| Sample C | 88% | 2.344% | 28.907% | 56.252% | 0.448% |

### Plasticizers

The oral product 110 can also include one or more plasticizers. Plasticizers can soften the final oral product and thus increase its flexibility. Plasticizers work by embedding themselves between the chains of polymers, spacing them apart (increasing the "free volume"), and thus significantly lowering the glass transition temperature for the plastic and making it softer. Suitable plasticizers include propylene glycol, glycerin, vegetable oil, and medium chain triglycerides. In some embodiments, the plasticizer can include phthalates. Esters of polycarboxylic acids with linear or branched aliphatic alcohols of moderate chain length can also be used as plasticizers. Moreover, plasticizers can facilitate the extrusion processes described below. In some embodiments, the oral product 110 can include up to 20 weight percent plasticizer. In some embodiments, the oral product 110 includes between 0.5 and 10 weight percent plasticizer, the oral product 110 can include between 1 and 8 weight percent plasticizer, or between 2 and 4 weight percent plasticizer. For example, an oral product comprising a polyurethane polymer matrix and include about 3 to 6.5 weight percent of propylene glycol.

### Molding Processes

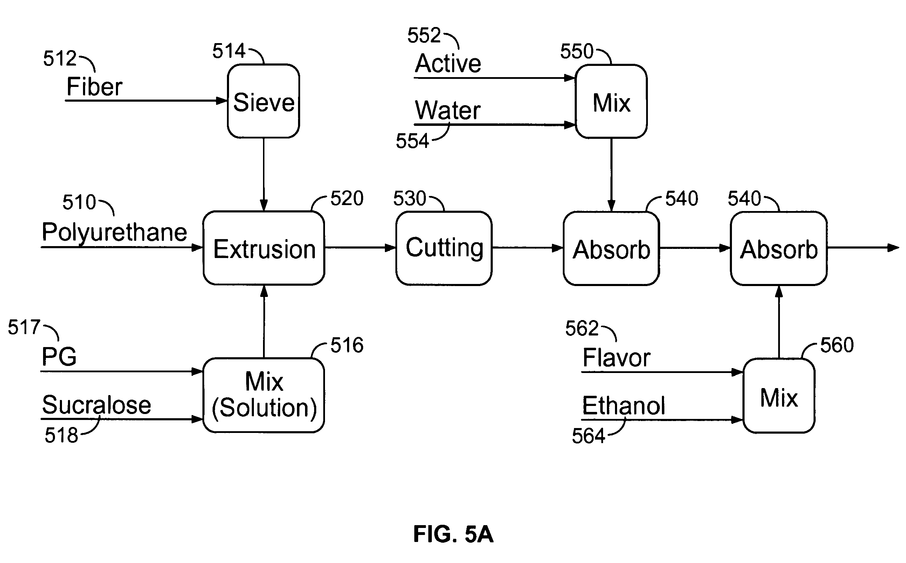

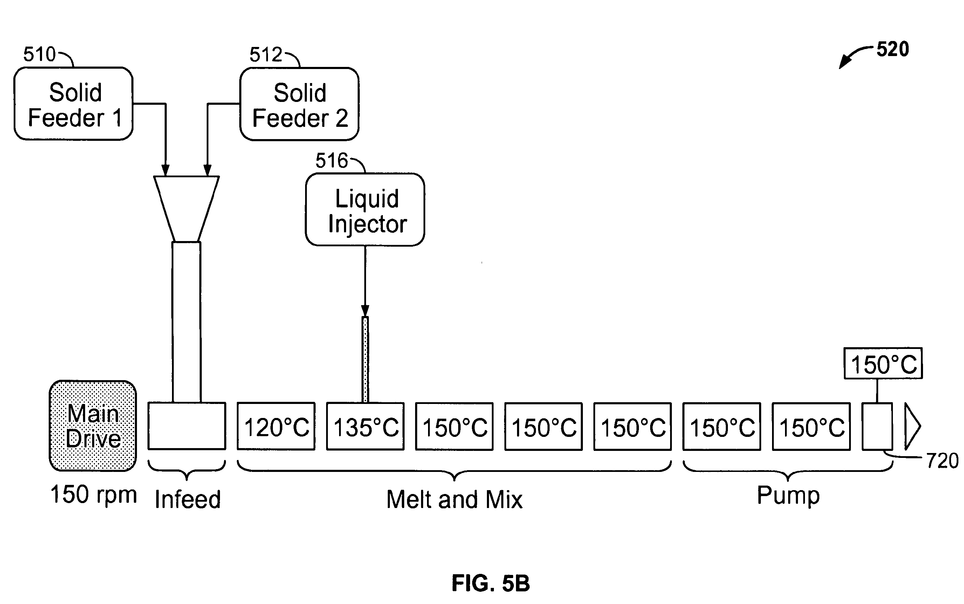

The oral product 110 can be produced by extruding a mouth-stable polymer (e.g., polyurethane) with fibers (e.g., cellulosic fiber) and/or additive (e.g., nicotine) to form a rod of a mouth-stable polymer matrix including fibers and/or additives. The rod is cut into individual oral products 110. Figures 5A and 5B depict exemplary methods to form oral products 110.

Referring to the extrusion process illustrated in Figure 5A, a mouth-stable polymer 510 (e.g., polyurethane) is introduced into an extruder for extrusion 520 along with fibers 512 (e.g., cellulosic fibers). The fibers 512 can be passed through a sieve 514 prior to introduction into the extruder. A mixture of additives 516 can also be introduced into the extruder. The mixture of additives 516 can be a solution (as shown). As shown, the additives can include a plasticizer 517 (e.g., propylene glycol) and a sweetener 518 (e.g., sucralose). The mixture of additives can also be provided in slurry form or a dry mix of powdered additives.

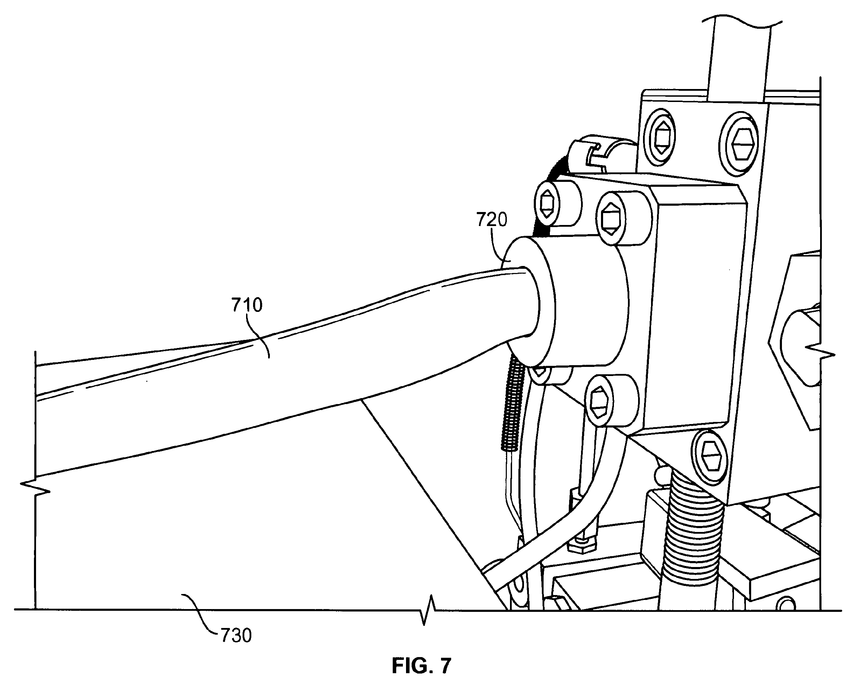

Figure 5B illustrates an example of how the mouth-stable polymer 510 (e.g., polyurethane) can be compounded with fiber 512 and a mixture of additives 516. As shown, polyurethane pellets 510 and cellulosic fibers 512 can be introduced into an infeed section of an extruder. A first section of the extruder melts and mixes the polymer, elevating the temperature to about 150 °C. The mixture 516 of propylene glycol 517 and sucralose 518 can be injected into the extruder downstream of the infeed section of the extruder. The
polymer/fiber/plasticizer/sweetener mixture can then be extruded out of an extrusion die 720 at a temperature of about 150°C. An example of an extrusion die is shown in Figure 7. For example, the extruder of Figure 5B can operate at a mass flow rate of about 1.8 lbs/hour.

The polymer-fiber combination can exit an extrusion die 720 as a rod 710 and onto a moving conveyor 730, as shown in Figure 7. The size of the extrusion die 720, the take away speed of the moving conveyor 730, the mixture of polymer-fiber combination, and the temperature of the mixture exiting the die 720 can all have an impact on the final diameter of the rod 710.

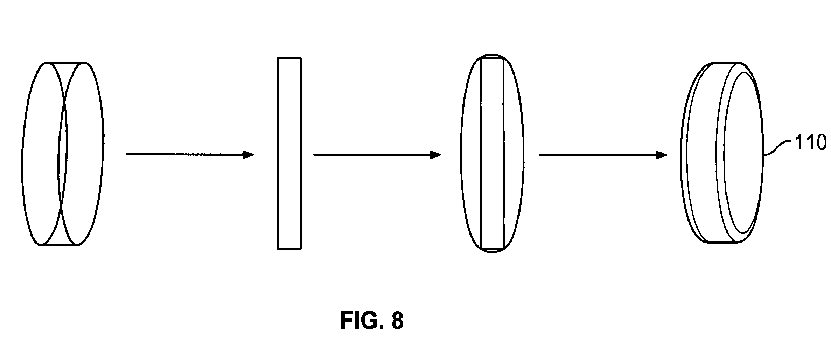

The extruded polymer-fiber rod 710 is then cut in a cutting process 530, as shown in Figure 5A. The cutting can be hot-face cutting. Hot-face cutting can occur immediately after the rod 720 exits the extrusion die 720. The cutting can induce pillowing of the polymer matrix, as shown in Figure 8. The cutting process 530 can also include a process of rounding the edges of the cut polymer-fiber composite. For example, a pelletizer can be used to round the edges. The pelletizer can also help to cool the oral products 110. In other embodiments, the extruded polymer-fiber rod 710 is cooled prior to cutting.

Before or after cutting, additional additives and/or flavorants can be added to the extruded polymer-fiber rod and/or pieces. As shown in Figure 5A, a mixture of additives 550 and a mixture of flavorants 560 can be absorbed into polymer-fiber pieces in one or more absorbing processes 540. The mixture of additives 550 can include 552 and water 554. A mixture of flavorants 560 can include a flavor 562 (e.g., wintergreen) and a carrier 564 (e.g., ethanol). The oral products 110 could then be dried, packaged, and sealed.

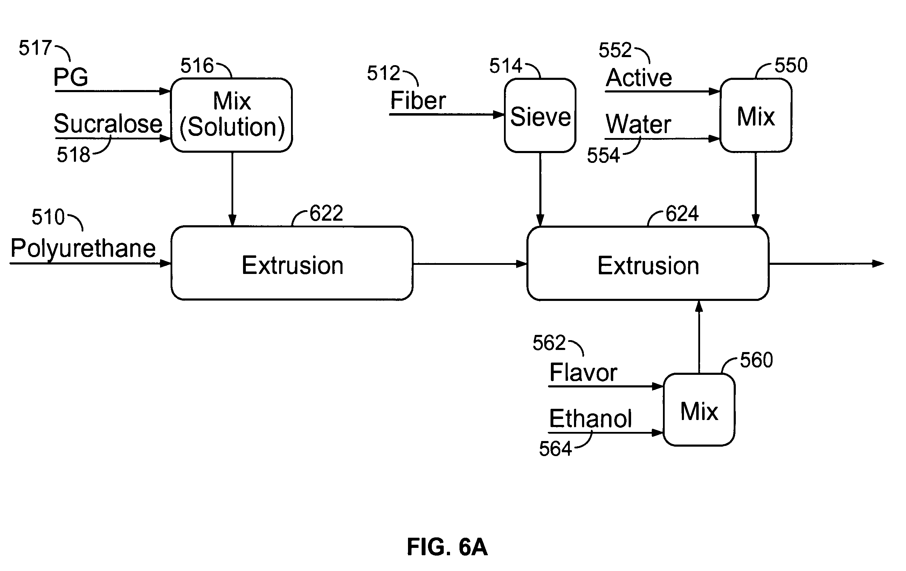

Figure 6A depicts an alternative arrangement where a mouth-stable polymer 510 (e.g., polyurethane) is compounded with a mixture 516 of one or more plasticizers 517 (e.g., propylene glycol) and/or sweeteners 518 (e.g., sucralose) in a first extrusion process 622. The compounded polymer/plasticizer/sweetener mixture is then compounded with fiber 512 in a second extrusion process 624. As shown, additives such as nicotine 552 and/or flavorants 562 can also be added during the second extrusion process 624. In some embodiments, the compounding in the first extrusion process occurs at a higher temperature than the compounding during the second extrusion process. Both extrusion processes can occur in a single extruder.

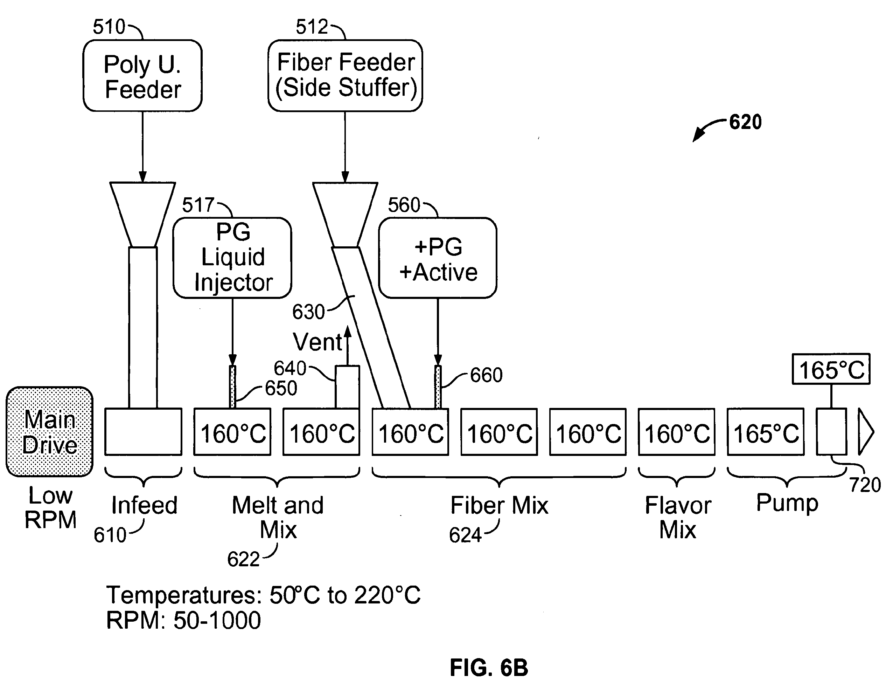

Figure 6B depicts an arrangement of an extruder where the active, plasticizer, fibers and flavorants are all added the mouth-stable polymer in the extruder. Polyurethane pellets 510 are added to an infeed section 610 of the extruder 620. Plasticizer 517 (e.g., propylene glycol) (and optionally actives, sweeteners, and/or carriers) are injected into a first section of the extruder and compounded with the polyurethane. A vent 640 can be provided to release volatiles. Cellulosic fibers 512 can be introduced into the extruder through a side feeder 630. A flavorant mixture 560 can be added through liquid injector 660 in a flavor mixing section of the extruder. Active 52 (e.g., nicotine) and plasticizer 517 can also be injected through liquid injector 660. The mixture can then be extruded through an extrusion die 720 at a temperature of about 165°C. The extruded mixture can be hot-cut as it exits the extrusion die 720 and passed to a pelletizer. In other embodiments, the extruded mixture can be cooled on a cooling conveyer and cut. For example, the extruder of Figure 6B can operate at a mass flow rate of about 5.5 lbs/hour. After cutting, the oral products 110 can be further flavored in a pan coater. The oral products 110 can then be sent to bulk storage and packaged.

In addition to the methods described above, there are many methods for making and shaping the oral products. In some embodiments, extruded and cut pieces can be introduced into a compression mold to form a final oral product shape. In other embodiments, the oral product 110 can be injection molded, compression molded, or injection-compression molded. Blocks of polymer, fiber, and/or additive can also be formed and machined into a desired shape.

### Examples

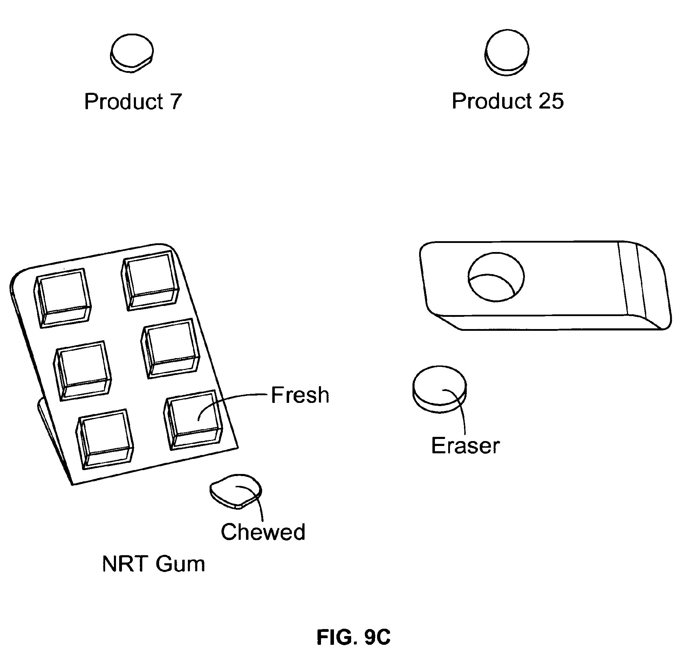

A series of oral products were produced by extruding a combination of polyurethane, cellulosic fiber, nicotine, propylene glycol, sweeteners, and flavorants to produce a variety of oral products, including the oral products listed in Table IV below. Each product sample was cut to have a thickness of about 3 mm. Each oral product (Products 7, 15, 18, 25, and 26) was subjected to the compression @ 250 N test, the compression @ 425 N test, and the springiness test discussed above. To compare the properties of the oral products 110, samples of nicotine replacement therapy (NRT) chewing gum¹ and a cut piece of a standard eraser² were also tested. The NRT gum was tested in both its natural state and after being chewed. A chewed sample of Product 25 was also tested. Figure 9C depicts some of the samples. As shown in the chart and in Figures 9A and 9B, the oral products were less compressive than the chewing gum and the eraser. During the compression tests of the NRT gum, each sample was punctured by the probe, thus they are indicated as having a 100% compression. The eraser was also punctured by the compression @ 425 N test. The oral products, however, each withstood the compression tests. The non-zero values for the compression tests of the oral products, however, indicate that the oral products are pliable and can be worked within the mouth.

**TABLE IV**

| **Analysis Date** | **Sample Number** | **Sample ID** | **Sample Thickness (mm)** | **Compression @ 250N (%)** | **Compression @ 425N (%)** | **Springiness (%)** |
|---|---|---|---|---|---|---|
| 10/4/2011 | 1 | **Product 7** | 3.04 | 49.65 | 69.21 | 86.61 |
| | 2 | **Product 15** | 2.95 | 60.17 | 83.09 | 82.10 |
| | 3 | **Product 18** | 3.01 | 59.44 | 82.95 | 83.01 |
| | 4 | **Product 25** | 3.02 | 61.92 | 84.91 | 82.27 |
| | 5 | **Product 26** | 3.04 | 72.10 | 93.39 | 81.84 |
| 10/11/2011 | 6 | **Product 25** - **"chewed"** | 2.88 | 78.14 | 95.27 | 86.32 |
| | 7 | **NRT Gum** | 2.63 | 100* | 100* | 15.82 |
| | 8 | **NRT Gum - "chewed"** | 2.72 | 100* | 100* | 7.57 |
| | 9 | **Eraser** | 2.94 | 87.52 | 100* | 80.82 |

| | | | | | | |
|---|---|---|---|---|---|---|
| * Sample was fully compressed by the probe (resulting in a hole in the sample) | | | | | | |

The oral products also demonstrated resilience in the springiness test discussed above. As shown, the NRT gum (both chewed and fresh) resulted in very little recovery after compression. The eraser, on the other hand, demonstrated a similar amount of springiness as compared to the oral products. These tests also demonstrate that the chewed oral product results in a more compressible product.

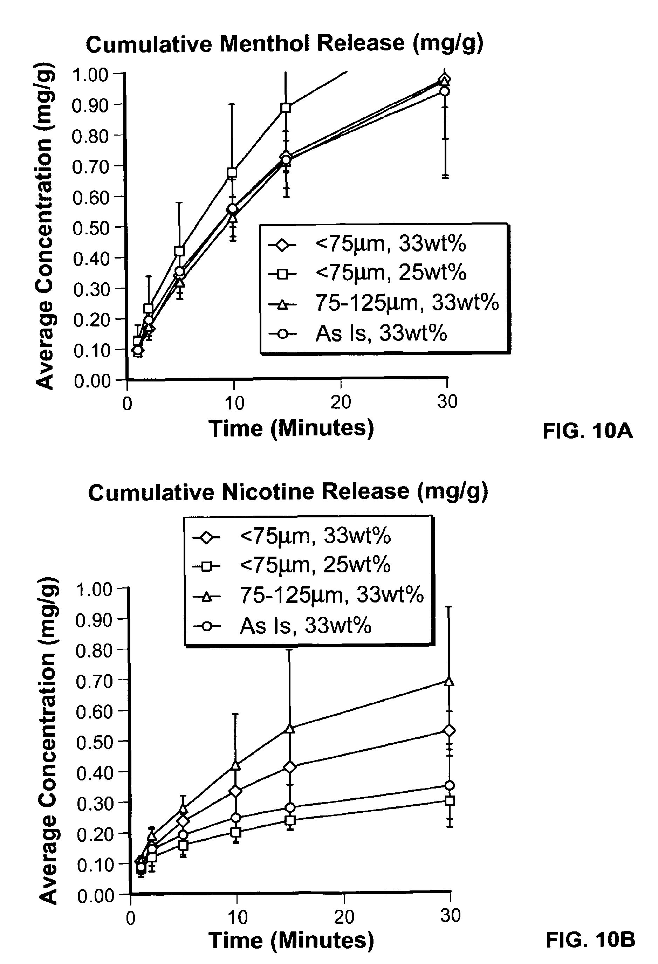

A series of oral products were also tested in a mastication test to determine the additive release profile. The results of this test are shown in Figures 10A and 10B. Each sample included polyurethane, cellulosic fiber, peppermint oil, and nicotine. The samples included about 2 mg of nicotine and about 1.6% total weight of peppermint oil. One constituent of peppermint oil is menthol. Peppermint oil may contain between 16 weight percent and 50 weight percent of menthol, usually about 30 weight percent, thus sampling the release of menthol is an appropriate way to approximate the release of peppermint oil. The different samples included different amounts of polymer (polyurethane), different amounts of cellulosic fiber, or different fiber sizes. Some of the samples had 59.70% polyurethane and 33.17% fiber, while other samples had 67.87% polyurethane and 25.00% fiber. Moreover, some of the samples had fiber having a size of less than 75 micrometers, some has a fiber size of between 75 and 125 micrometers, and some included bulk fiber. The samples also included nicotine and menthol (as a flavorant). Each sample was placed in a mastication tester that manipulated the sample in a solution that mimics saliva. At varying time intervals, samples were taken from the mastication tester to determine the total amount of menthol and nicotine released. As shown in Figures 10A and 10B, each sample continued to have a release of the additives even after 15 minutes of mastication. As compared to chewing gums, this is an increased additive release time period.

The release of menthol was accelerated in the sample having a higher percentage of polyurethane and a lower percentage of fiber. The release of the nicotine was accelerated by having a higher percentage of fiber and by having larger fiber sizes. Accordingly, the release rate of water-soluble additives may be determined by the amount of fibers and by the fiber dimensions in the oral product.

### Other Embodiments

Disclosed are methods and compositions that can be used for, can be used in conjunction with, can be used in preparation for, or are products of the disclosed methods and compositions. These and other materials are disclosed herein, and it is understood that combinations, subsets, interactions, groups, etc. of these methods and compositions are disclosed. That is, while specific reference to each various individual and collective combinations and permutations of these compositions and methods may not be explicitly disclosed, each is specifically contemplated and described herein. For example, if a particular composition of matter or a particular method is disclosed and discussed and a number of compositions or methods are discussed, each and every combination and permutation of the compositions and the methods are specifically contemplated unless specifically indicated to the contrary. Likewise, any subset or combination of these is also specifically contemplated and disclosed.

## Claims

1. An oral product,
(A) comprising a body that is wholly receivable in an oral cavity, the body comprising:
a mouth-stable polymer matrix;
cellulosic fibers embedded in the mouth-stable polymer matrix; and
nicotine or derivative thereof dispersed in the mouth-stable polymer matrix such that the nicotine or derivative thereof is released from the body when the body is received within the oral cavity and exposed to saliva;
(B) comprising a body that is wholly receivable in an oral cavity, the body comprising:
a mouth-stable polymer matrix;
cellulosic fibers embedded in the mouth-stable polymer matrix; and
an additive dispersed in the mouth-stable polymer matrix such that the additive is released from the body when the body is received within the oral cavity and exposed to saliva;
(C) comprising a body that is wholly receivable in an oral cavity, the body comprising:
a mouth-stable polymer matrix;
exhausted tobacco fibers embedded in the mouth-stable polymer matrix; and
an additive dispersed in the mouth-stable polymer matrix such that the additive is released from the body when the body is received within the oral cavity and exposed to saliva;
(D) which is an oral tobacco product, comprising a body that is wholly receivable in an oral cavity, the body comprising:
a mouth-stable polymer matrix; and
tobacco fibers embedded in the mouth-stable polymer matrix,
wherein the body has a compressibility @ 250 N of between 45% and 95%;
(E) which is an oral tobacco product, comprising a body that is wholly receivable in an oral cavity, the body comprising:
a mouth-stable polymer matrix; and
tobacco fibers embedded in the mouth-stable polymer matrix,
wherein the body has a compressibility @ 425 N of between 60% and 99%; or
(F) which is an oral tobacco product, comprising a body that is wholly receivable in an oral cavity, the body comprising:
a mouth-stable polymer matrix; and
tobacco fibers embedded in the mouth-stable polymer matrix,
wherein the body has a percentage of springiness of at least 20%;
wherein in (A)-(F) above,
the mouth-stable polymer matrix includes a polymer selected from the group consisting of polyurethanes, silicone polymers, polyesters, polyacrylates, polyethylenes, polypropylenes, polyetheramides, polystyrenes, polyvinyl alcohols, polyvinyl acetates, polyvinyl chlorides, polybutyl acetates, butyl rubbers, and mixtures, combinations and copolymers thereof, and wherein the mouth-stable polymer matrix does not appreciably dissolve or disintegrate when exposed to saliva at about 37°C (about 98.6°F) over a period of one hour; and
wherein cellulosic fibers include cellulose or treated cellulose material.

2. The oral product of claim 1 (A),
(i) wherein the mouth-stable polymer matrix comprises polyurethane, and wherein preferably the nicotine is synthetic nicotine;
(ii) wherein the mouth-stable polymer matrix comprises polyester, and wherein preferably the nicotine is synthetic nicotine;
(iii) wherein the mouth-stable polymer matrix comprises polyacrylate, and wherein preferably the nicotine is synthetic nicotine;
(iv) wherein the mouth-stable polymer matrix comprises polyethylene, SEBS, SBS, or a combination thereof, and wherein preferably the nicotine is synthetic nicotine;
(v) further comprising a plasticizer dispersed in the mouth-stable polymer matrix, and wherein preferably the nicotine is synthetic nicotine, wherein the plasticizer is preferably selected from the group consisting of propylene glycol, glycerin, vegetable oil, triglycerides, and combinations thereof;
(vi) further comprising a sweetener dispersed in the body, and wherein preferably the nicotine is synthetic nicotine, wherein the sweetener is preferably selected from the group consisting of saccharine, sucralose, aspartame, acesulfame potassium, and combinations thereof;
(vii) further comprising an additive selected from the group consisting of minerals, vitamins, dietary supplements, nutraceuticals, energizing agents, soothing agents, amino acids, chemsthetic agents, antioxidants, botanicals, teeth whitening agents, therapeutic agents, and combinations thereof, wherein the additive is dispersed in the body such that the additive is released when the body is held within a mouth of an adult consumer;
(viii) further comprising a flavorant dispersed in the body such that the flavorant is released when the body is held within a mouth of an adult consumer, wherein the flavorant is preferably selected from the group consisting of licorice, wintergreen, cherry and berry type flavorants, Dramboui, bourbon, scotch, whiskey, spearmint, peppermint, lavender, cinnamon, cardamon, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, anise, coriander, coffee, mint oils from a species of the genus Mentha, and combinations thereof;
(ix) wherein the nicotine is tobacco-derived nicotine;
(x) wherein the nicotine is synthetic nicotine;
(xi) wherein the oral product is substantially free of tobacco plant tissue;
(xii) wherein the nicotine is absorbed in the fiber-polymer matrix;
(xiii) wherein the body is shield shaped;
(xiv) wherein the body has a diameter of between 5 mm and 25 mm and a thickness of between 1 mm and 10 mm;
(xv) wherein the body comprises at least 10 weight percent cellulosic fibers;
(xvi) wherein the cellulosic fibers are non-tobacco cellulosic fibers, preferably the cellulosic fibers are sugar beet fibers, wood pulp fiber, cotton fiber, bran fiber, citrus pulp fiber, grass fiber, willow fiber, and poplar fiber;
(xvii) wherein the body comprises at least 10 weight percent of the mouth-stable polymer;
(xviii) wherein the body comprises between 0.1 mg and 6 mg nicotine;
(xix) wherein the body has a compressibility @ 250 N of less than 95%;
(xx) wherein the body has a compressibility @ 250 N of less than 80%;
(xxi) wherein the body has a compressibility @ 250 N of between 45% and 90%;
(xxii) wherein the body has a compressibility @ 425 N of less than 99%;
the body has a compressibility @ 425 N of between 60% and 98%;
(xxiii) wherein the body has a percentage of springiness of at least 20%;
(xxiv) wherein the body has a percentage of springiness of at least 70%;
(xxv) wherein the body has a percentage of springiness of between 75% and 90%;
(xxvi) wherein the oral product further comprises an antioxidant; wherein the oral product preferably comprises between 0.01 weight percent and 5.0 weight percent antioxidant; or wherein the antioxidant is preferably selected from ascorbyl palmitate, BHT, ascorbic acid, sodium ascorbate, monosterol citrate, tocopherols, propyl gallate, tertiary butylhydroquinone (TBHQ), butylated hydroxyanisole (BHA), Vitamin E, and combination thereof; or wherein the antioxidant is a derivative of Vitamin C; or wherein the antioxidant is ascorbyl palmitate; or
(xxvii) further comprising an antioxidant and a soluble fiber, wherein the ratio of soluble fiber to cellulosic fibers is preferably between 1:60 and 60:1; or wherein the soluble fibers comprise maltodextrin; or wherein the soluble fibers are derived from corn.

3. A method of forming an oral product according to claim 1 comprising:
(A) extruding a mouth-stable polymer having cellulosic fibers dispersed therein; and dispersing nicotine or a derivative thereof within the mouth-stable polymer during or after the extruding step;
(B) extruding a mouth-stable polymer;
dispersing nicotine within the mouth-stable polymer during or after the extruding step; and
cutting the extruded mouth-stable polymer to form individual oral products;
(C) extruding a mouth-stable polymer;
dispersing nicotine within the mouth-stable polymer during or after the extruding step; and
forming the extruded mouth-stable polymer to form individual oral products;
(D) extruding a mouth-stable polymer having cellulosic fibers dispersed therein; and dispersing an additive within the mouth-stable polymer during or after the extruding step, the additive being selected from a flavorant, a sweetener, caffeine, or a combination thereof;
(E) extruding a mouth-stable polymer;
dispersing an additive within the mouth-stable polymer during or after the extruding step; and
cutting the extruded mouth-stable polymer to form individual oral products;
(F) extruding a mouth-stable polymer;
dispersing an additive within the mouth-stable polymer during or after the extruding step; and
forming the extruded mouth-stable polymer to form individual oral products;
(G) extruding a mouth-stable polymer having exhausted tobacco fibers dispersed therein; and dispersing an additive within the mouth-stable polymer during or after the extruding step, the additive being selected from a flavorant, a sweetener, nicotine, caffeine, or a combination thereof; or
(H) wherein the oral product is an oral tobacco product and the method comprises:
extruding a mouth-stable polymer having tobacco fibers dispersed therein; and
cutting the extruded mixture of mouth-stable polymer and tobacco fibers into individual oral tobacco products sized to be at least partially received in an oral cavity of an adult tobacco consumer.

4. The method of claim 3(A),
(i) further comprising cutting the extruded mixture of mouth-stable polymer and cellulosic fibers into individual oral products sized to be at least partially received in an oral cavity of an adult consumer;
(ii) wherein the dispersing step occurs during the extruding step, wherein the nicotine is preferably mixed with the cellulosic fibers;
(iii) wherein the dispersing step occurs after the extruding step;
(iv) wherein the mouth-stable polymer comprises a plasticizer mixed therein during the extruding step;
(v) wherein the method further comprises adding a flavorant during or after the extruding step;
(vi) wherein the method further comprises adding an antioxidant during the extruding step; or
(vii) wherein the method further comprises adding an antioxidant during a post-extrusion flavoring process; or
(viii) wherein the method further comprises adding soluble fibers during the extruding step.

5. The method of claim 3(B), wherein the individual oral products are pliable and have shape memory when manipulated within an oral cavity.

6. The oral product of claim 1(A), wherein
(i) the body comprises between 0.01 weight percent and 1.0 weight percent antioxidant and between 0.1 mg and 6 mg nicotine;
(ii) an antioxidant is dispersed in the body, wherein the antioxidant is ascorbyl palmitate, BHT, ascorbic acid, sodium ascorbate, a derivative of Vitamin C, monosterol citrate, tocopherols, propyl gallate, tertiary butylhydroquinone (TBHQ), butylated hydroxyanisole (BHA), Vitamin E, and derivative or combination thereof; or
(iii) the body comprises a combination of soluble fibers and insoluble cellulosic fibers within the matrix, wherein the nicotine and the antioxidant are dispersed within the matrix.

7. The oral product of claim 1(B),
(i) wherein the mouth-stable polymer matrix comprises polyurethane;
(ii) further comprising a sweetener dispersed in the body;
(iii) wherein the additive is selected from the group consisting of minerals, vitamins, dietary supplements, nutraceuticals, energizing agents, soothing agents, amino acids, chemsthetic agents, antioxidants, botanicals, teeth whitening agents, therapeutic agents, and combinations thereof;
(iv) wherein the additive is a therapeutic agent selected from the group consisting of Gerd, Buprenorphin, Nitroglycerin, Diclofenac, Fentanyl, Carbamazepine, Galantamine, Acyclovir, Polyamidoamine Nanoparticles, Chlorpheniramine, Testosterone, Estradiol, Progesterone, Calcitonin, Fluorouracil, Naltrexone, Odansetron, Decitabine, Selegiline, Lamotrigine, Prochlorperazine, and combinations thereof;
(v) further comprising a flavorant dispersed in the body such that the flavorant is released when the body is held within a mouth of a consumer;
(vi) wherein the body has a compressibility @ 250 N of between 45% and 90%;
(vii) wherein the body has a compressibility @ 425 N of between 60% and 98%; or
(viii) wherein the body has a percentage of springiness of between 75% and 90%.

8. The method of claim 3(E), wherein the individual oral products have a compressibility @ 250 N of between 45% and 90%.

9. The method of claim 3(F), wherein the individual oral products have a compressibility @ 250 N of between 45% and 90%.

10. The oral product of claim 1(C),
(i) wherein the mouth-stable polymer matrix comprises a polyurethane;
(ii) further comprising a sweetener dispersed in the body;
(iii) wherein the additive is nicotine or a derivative thereof;
(iv) wherein the oral product further comprises non-tobacco cellulosic fibers;
(v) wherein the additive is selected from the group consisting of minerals, vitamins, dietary supplements, nutraceuticals, energizing agents, soothing agents, amino acids, chemsthetic agents, antioxidants, botanicals, teeth whitening agents, therapeutic agents, and combinations thereof; or
(vi) further comprising a flavorant dispersed in the body or exhausted tobacco fibers such that the flavorant is released when the body is held within a mouth of an adult consumer.

11. The oral product of claim 1(C), wherein
(i) the body comprises at least 10 weight percent exhausted tobacco fibers;
(ii) the body comprises at least 10 weight percent of the mouth-stable polymer;
(iii) the body comprises between 0.1 mg and 6 mg nicotine;
(iv) the body has a compressibility @ 250 N of between 45% and 90%;
(v) the body has a compressibility @ 425 N of between 60% and 98%; or
(vi) the body has a percentage of springiness of between 75% and 90%.

12. The oral tobacco product of one of claims 1(D)-(F),
(i) wherein the mouth-stable polymer matrix comprises polyurethane;
(ii) further comprising a plasticizer dispersed in the mouth-stable polymer matrix;
(iii) wherein the body is shield shaped;
(iv) wherein the body comprises at least 10 weight percent tobacco fibers;
(v) further comprising non-tobacco cellulosic fibers;
(vi) wherein the body comprises at least 10 weight percent of the mouth-stable polymer;
(vii) wherein the body has a compressibility @ 250 N of between 45% and 90%;
(viii) wherein the body has a compressibility @ 425 N of between 60% and 98%;
(ix) wherein the body has a percentage of springiness of between 75% and 90%;
(x) further comprising an antioxidant, preferably, wherein the oral product comprises between 0.01 weight percent and 5.0 weight percent antioxidant; or
(xi) further comprising a soluble fiber, preferably, wherein the ratio of soluble fiber to cellulosic fibers is between 1:60 and 60:1, or wherein the soluble fibers comprise maltodextrin.

13. The method of claim 3(H), wherein the method further comprises adding soluble fibers during the extruding step.

## Patentansprüche

1. Orales Produkt,
(A) umfassend einen Körper, der vollständig in eine Mundhöhle aufnehmbar ist, wobei der Körper umfasst:
eine Mund-stabile Polymermatrix;
Zellulosefasern, eingebettet in diese Mund-stabile Polymermatrix; und
Nikotin oder ein Derivat davon, dispergiert in der Mund-stabilen Polymermatrix, derart dass das Nikotin oder Derivat davon aus dem Körper freigesetzt wird, wenn der Körper innerhalb der Mundhöhle aufgenommen und gegenüber Speichel exponiert ist;
(B) umfassend einen Körper, der vollständig in eine Mundhöhle aufnehmbar ist, wobei der Körper umfasst:
eine Mund-stabile Polymermatrix;
Zellulosefaser, eingebettet in die Mund-stabile Polymermatrix; und
ein Additiv, dispergiert in der Mund-stabilen Polymermatrix, derart dass das Additiv aus dem Körper freigesetzt wird, wenn der Körper innerhalb der Mundhöhle aufgenommen und gegenüber Speichel exponiert ist;
(C) umfassend einen Körper, der vollständig in eine Mundhöhle aufnehmbar ist, wobei der Körper umfasst:
eine Mund-stabile Polymermatrix;
ausgelaugte (exhausted) Tabakfasern, eingebettet in der Mund-stabilen Polymermatrix; und
ein Additiv, dispergiert in der Mund-stabilen Polymermatrix, derart dass das Additiv aus dem Körper freigesetzt wird, wenn der Körper innerhalb der Mundhöhle aufgenommen und gegenüber Speichel exponiert ist;
(D) welches ein orales Tabakprodukt ist, umfassend einen Körper, der vollständig in eine Mundhöhle aufnehmbar ist, wobei der Körper umfasst:
eine Mund-stabile Polymermatrix; und
Tabakfasern, eingebettet in der Mund-stabilen Polymermatrix,
wobei der Körper eine Kompressibilität @ 250 N von zwischen 45% und 95% aufweist;
(E) welcher ein orales Tabakprodukt ist, umfassend einen Körper, der vollständig in eine Mundhöhle aufnehmbar ist, wobei der Körper umfasst:
eine Mund-stabile Polymermatrix; und
Tabakfasern, eingebettet in die Mund-stabile Polymermatrix,
wobei der Körper eine Kompressibilität @ 425 N von zwischen 60% und 99% aufweist; oder
(F) welcher ein orales Tabakprodukt ist, umfassend einen Körper, der vollständig in eine Mundhöhle aufnehmbar ist, wobei der Körper umfasst:
eine Mund-stabile Polymermatrix; und
Tabakfasern, eingebettet in der Mund-stabilen Polymermatrix,
wobei der Körper einen Prozentsatz an Elastizität von mindestens 20% aufweist;
wobei in (A)-(F) oben,
die Mund-stabile Polymermatrix ein Polymer einschließt, ausgewählt aus der Gruppe bestehend aus Polyurethanen, Silikonpolymeren, Polyestern, Polyacrylaten, Polyethylenen, Polypropylenen, Polyetheramiden, Polystyrolen, Polyvinylalkoholen, Polyvinylacetaten, Polyvinylchloriden, Polybutylacetaten, Butylkautschuken, und Mischungen, Kombinationen und Co-polymeren davon, und wobei die Mund-stabile Polymermatrix sich nicht in nennenswerter Weise auflöst oder desintegriert, wenn sie gegenüber Speichel bei etwa 37°C (etwa 98,6°F) über einen Zeitraum von einer Stunde exponiert ist; und wobei die Zellulosefasern Zellulose oder behandeltes Zellulosematerial einschließen.

2. Das orale Produkt nach Anspruch 1(A),
(i) wobei die Mund-stabile Polymermatrix Polyurethan umfasst, und wobei vorzugsweise das Nikotin synthetisches Nikotin ist;
(ii) wobei die Mund-stabile Polymermatrix Polyester umfasst, und wobei vorzugsweise das Nikotin synthetisches Nikotin ist;
(iii) wobei die Mund-stabile Polymermatrix Polyacrylat umfasst, und wobei vorzugsweise das Nikotin synthetisches Nikotin ist;
(iv) wobei die Mund-stabile Polymermatrix Polyethylen, SEBS, SBS oder eine Kombination davon umfasst, und wobei das Nikotin vorzugsweise synthetisches Nikotin ist;
(v) weiterhin umfassend einen Weichmacher, dispergiert in der Mund-stabilen Polymermatrix, und wobei vorzugsweise das Nikotin synthetisches Nikotin ist, wobei der Weichmacher vorzugsweise ausgewählt ist aus der Gruppe bestehend aus Propylenglykol, Glycerin, Pflanzenöl, Triglyceriden und Kombinationen davon;
(vi) weiterhin umfassend ein Süßungsmittel, dispergiert in dem Körper, und wobei vorzugsweise das Nikotin synthetisches Nikotin ist, wobei das Süßungsmittel bevorzugt ausgewählt ist aus der Gruppe bestehend aus Saccharin, Sucralose, Aspartam, Acesulfam Kalium, und Kombinationen davon;
(vii) weiterhin umfassend ein Additiv, ausgewählt aus der Gruppe bestehend aus Mineralien, Vitaminen, Nahrungsergänzungsmitteln, Neutraceutical, belebenden Mitteln, lindernden Mitteln, Aminosäuren, chemosthetischen Mitteln (chemsthetic agents), Antioxidantien, Pflanzenstoffen, Zähne-weiß-machenden Mitteln, therapeutischen Mitteln, und Kombinationen davon, wobei das Additiv in dem Körper dispergiert ist, derart dass das Additiv freigesetzt wird, wenn der Körper innerhalb des Mundes eines erwachsenen Konsumenten behalten wird;
(viii) weiterhin umfassend einen Geschmacksstoff, dispergiert in dem Körper, derart dass der Geschmackstoff freigesetzt wird, wenn der Körper innerhalb des Mundes eines erwachsenen Konsumenten behalten wird, wobei der Geschmacksstoff bevorzugt ausgewählt ist aus der Gruppe bestehend aus Süßholzwurzel, Wintergrün, Kirsch- und Beeren typische Geschmacksstoffe, Drambuie, Bourbon, Scotch, Whiskey, grüne Minze, Pfefferminz, Lavendel, Zimt, Kardamon, Sellerie, Gewürznelke, Cascarilla, Muskat, Sandelholz, Bergamotte, Geranie, Honigessenz, Rosenöl, Vanille, Zitronenöl, Orangenöl, japanische Minze, Kassia, Kümmel, Cognac, Jasmin, Kamille, Menthol, Ylang-Ylang, Salbei, Fenchel, Pimenta, Ingwer, Anis, Koriander, Kaffee, Minzöle aus einer Spezies der Gattung der Minzen, und Kombinationen davon;
(ix) wobei das Nikotin ein Tabak abgeleitetes Nikotin ist;
(x) wobei das Nikotin synthetisches Nikotin ist;
(xi) wobei das orale Produkt im Wesentlichen frei von Tabakpflanzengewebe ist;
(xii) wobei das Nikotin in der Faser-Polymermatrix absorbiert ist;
(xiii) wobei der Körper schildförmig geformt ist;
(xiv) wobei der Körper einen Durchmesser von zwischen 5 mm und 25 mm und eine Dicke von zwischen 1 mm und 10 mm aufweist;
(xv) wobei der Körper mindestens zehn Gewichtsprozent Zellulosefasern umfasst;
(xvi) wobei die Zellulosefasern Nicht-Tabak-Zellulosefasern sind, bevorzugt sind die Zellulosefasern Zuckerrübenfasern, Zellstofffaser, Baumwollfaser, Kleiefaser, Zitruspulpenfaser, Grasfaser, Weidenfaser, und Pappelfaser;
(xvii) wobei der Körper mindestens zehn Gewichtsprozent des Mund-stabilen Polymers umfasst;
(xviii) wobei der Körper zwischen 0,1 mg und 6 mg Nikotin umfasst;
(xix) wobei der Körper eine Kompressibilität @ 250 N von weniger als 95% aufweist;
(xx) wobei der Körper eine Kompressibilität @ 250 N von weniger als 80% aufweist;
(xxi) wobei der Körper eine Kompressibilität @ 250 N von zwischen 45% und 90% aufweist;
(xxii) wobei der Körper eine Kompressibilität @ 425 N von weniger als 99% aufweist; wobei der Körper eine Kompressibilität @ 425 N von zwischen 60% und 98% aufweist;
(xxiii) wobei der Körper einen Prozentsatz an Elastizität von mindestens 20% aufweist;
(xxiv) wobei der Körper einen Prozentsatz an Elastizität von mindestens 70% aufweist;
(xxv) wobei der Körper einen Prozentsatz an Elastizität von zwischen 75% und 90% aufweist;
(xxvi) wobei das orale Produkt des Weiteren ein Antioxidationsmittel umfasst; wobei das orale Produkt bevorzugt zwischen 0,01 Gewichtsprozent und 5,0 Gewichtsprozent an Antioxidationsmittel umfasst; oder wobei das Antioxidationsmittel bevorzugt ausgewählt ist aus Ascorbin-Palmitat, BHT, Ascorbinsäure, Natriumascorbat, Monosterolcitrat, Tocopherolen, Propylgallat, tertiäres Butylhydrochinon (TBHQ), butyliertes Hydroxyanisol (BHA), Vitamin E, und Kombinationen davon; oder wobei das Antioxidationsmittel ein Derivat von Vitamin C ist; oder wobei das Antioxidationsmittel Ascorbin-Palmitat ist; oder (xxvii) weiterhin umfassend ein Antioxidationsmittel und eine lösliche Faser, wobei das Verhältnis der löslichen Faser zu den Zellulosefasern bevorzugt zwischen 1:60 und 60:1 beträgt; oder wobei die löslichen Fasern Maltodextrin umfassen; oder wobei die löslichen Fasern aus Mais erhalten sind.

3. Verfahren zum Bilden eines oralen Produkts gemäß Anspruch 1, umfassend:
(A) Extrudieren eines Mund-stabilen Polymers mit darin dispergierten Zellulosefasern; und Dispergieren von Nikotin oder einem Derivat davon innerhalb des Mund-stabilen Polymers während oder nach dem Extrudierschritt;
(B) Extrudieren eines Mund-stabilen Polymers;
Dispergieren von Nikotin innerhalb des Mund-stabilen Polymers während oder nach dem Extrudierschritt;
und
Schneiden des extrudierten Mund-stabilen Polymers um individuelle orale Produkte zu formen;
(C) Extrudieren eines Mund-stabilen Polymers;
Dispergieren von Nikotin innerhalb des Mund-stabilen Polymers während oder nach dem Extrudierschritt;
und
Formen des extrudierten Mund-stabilen Polymers, um individuelle orale Produkte zu formen;
(D) Extrudieren eines Mund-stabilen Polymers mit darin dispergierten Zellulosefasern; und Dispergieren eines Additivs innerhalb des Mund-stabilen Polymers während oder nach dem Extrudierschritt, wobei das Additiv ausgewählt ist aus einem Geschmacksstoff, einem Süßungsmittel, Koffein, oder einer Kombination davon;
(E) Extrudieren eines Mund-stabilen Polymers;
Dispergieren eines Additivs innerhalb des Mund-stabilen Polymers während oder nach dem Extrudierschritt; und
Schneiden des extrudierten Mund-stabilen Polymers, um individuelle orale Produkte zu formen;
(F) Extrudieren eines Mund-stabilen Polymers;
Dispergieren eines Additivs innerhalb des Mund-stabilen Polymers während oder nach dem Extrudierschritt;
und
Formen des extrudierten Mund-stabilen Polymers, um individuelle orale Produkte zu formen;
(G) Extrudieren eines Mund-stabilen Polymers mit darin dispergierten ausgelaugten (exhausted) Tabakfasern; und dispergieren eines Additivs innerhalb des Mund-stabilen Polymers während oder nach dem Extrudierschritt, wobei das Additiv ausgewählt ist aus einem Geschmacksstoff, einem Süßungsmittel, Nikotin, Koffein, oder einer Kombination davon; oder
(H) wobei das orale Produkt ein orales Tabakprodukt ist und das Verfahren umfasst:
Extrudieren eines mund-stabilen Polymers mit darin dispergierten Tabakfasern; und Schneiden der extrudierten Mischung des Mund-stabilen Polymers und Tabakfasern in individuelle orale Tabakprodukte, in der Größe bemessen, um zumindest teilweise in der Mundhöhle eines erwachsenen Tabakkonsumenten aufgenommen zu werden.

4. Das Verfahren nach Anspruch 3(A),
(i) weiterhin das Schneiden der extrudierten Mischung des Mund-stabilen Polymers und der zellulosehaltigen Fasern in individuelle orale Produkte, in der Größe bemessen, um zumindest teilweise in der Mundhöhle eines erwachsenen Konsumenten aufgenommen zu werden, umfassend;
(ii) wobei der Dispergierschritt während des Extrudierschritts erfolgt, wobei das Nikotin vorzugsweise mit den zellulosehaltigen Fasern gemischt wird;
(iii) wobei der Dispergierschritt nach dem Extrudierschritt erfolgt;
(iv) wobei das Mund-stabile Polymer einen Weichmacher, der während des Extrudierschritts dort hinein gemischt wird, umfasst;
(v) wobei das Verfahren des Weiteren das Zugeben eines Geschmacksstoffs während oder nach dem Extrudierschritt umfasst;
(vi) wobei das Verfahren weiterhin das Zugeben eines Antioxidationsmittels während des Extrudierschritts umfasst; oder
(vii) wobei das Verfahren des Weiteren umfasst das Zugeben eines Antioxidationsmittels während eines Nach-Extrusions-Aromatisierschrittes; oder
(viii) wobei das Verfahren weiterhin das Zugeben von löslichen Fasern während des Extrudierschritts umfasst.

5. Das Verfahren nach Anspruch 3(B), wobei die individuellen oralen Produkte flexibel sind und ein Formgedächtnis aufweisen, wenn sie innerhalb der Mundhöhle gehandhabt werden.

6. Das orale Produkt nach Anspruch 1(A), wobei
(i) der Körper zwischen 0,01 Gewichtsprozent und 1,0 Gewichtsprozent an Antioxidationsmittel und zwischen 0,1 mg und 6 mg Nikotin umfasst;
(ii) ein Antioxidationsmittel in dem Körper dispergiert ist, wobei das Antioxidationsmittel Ascorbylpalmitat, BHT, Ascorbinsäure, Natriumascorbat, ein Derivat von Vitamin C, Monosterolcitrat, Tocopherole, Propylgallat, tertiäres Butylhydrochinon (TBHQ), butyliertes Hydroxyanisol (BHA), Vitamin E, und ein Derivat oder eine Kombination davon ist; oder
(iii) der Körper eine Kombination von löslichen Fasern und unlöslichen Zellulosefasern innerhalb der Matrix umfasst, wobei das Nikotin und das Antioxidationsmittel innerhalb der Matrix dispergiert sind.

7. Das orale Produkt nach Anspruch 1(B),
(i) wobei die Mund-stabile Polymermatrix Polyurethan umfasst;
(ii) weiterhin umfassend ein Süßungsmittel, das in dem Körper dispergiert ist;
(iii) wobei das Additiv ausgewählt ist aus der Gruppe aus Mineralien, Vitaminen, Nahrungsergänzungsmitteln, Neutraceuticals, belebenden Mitteln, lindernden Mitteln, Aminosäuren, chemosthetischen Mitteln (chemsthetic agents), Antioxidantien, Pflanzenstoffen, Zähne-weiß-machenden Mitteln, therapeutischen Mitteln, und Kombinationen davon;
(iv) wobei das Additiv ein therapeutisches Mittel ist, ausgewählt aus der Gruppe bestehend aus Gerd, Buprenorphin, Nitroglycerin, Diclofenac, Fentanyl, Carbamazepin, Galantamin, Acyclovir, Polyamidoamin Nanopartikeln, Chlorpheniramine, Testosteron, Estradiol, Progesteron, Calcitonin, Fluorouracil, Naltrexon, Odansetron, Decitabin, Selegilin, Lamotrigin, Prochlorperazin, und Kombinationen davon;
(v) weiterhin umfassend einen Geschmacksstoff, der in dem Körper dispergiert ist, derart, dass der Geschmacksstoff freigesetzt wird, wenn der Körper innerhalb des Mundes eines Konsumenten behalten wird;
(vi) wobei der Körper eine Kompressibilität @ 250 N von zwischen 45% und 90% aufweist;
(vii) wobei der Körper eine Kompressibilität @ 425 N von zwischen 60% und 98% aufweist; oder
(viii) wobei der Körper einen Prozentsatz an Elastizität von zwischen 75% und 90% aufweist.

8. Das Verfahren nach Anspruch 3(E), wobei die individuellen oralen Produkte eine Kompressibilität @ 250 N von zwischen 45% und 90% aufweisen.

9. Das Verfahren nach Anspruch 3(F), wobei die individuellen oralen Produkte eine Kompressibilität @ 250 N von zwischen 45% und 90% aufweisen.

10. Das orale Produkt nach Anspruch 1(C),
(i) wobei die Mund-stabile Polymermatrix ein Polyurethan umfasst;
(ii) weiterhin umfassend ein Süßungsmittel, dispergiert in dem Körper;
(iii) wobei das Additiv Nikotin oder ein Derivat davon ist;
(iv) wobei das orale Produkt weiterhin Nicht-Tabak-Zellulosefasern umfasst;
(v) wobei das Additiv ausgewählt ist aus der Gruppe bestehend aus Mineralien, Vitaminen, Nahrungsergänzungsmitteln, Neutraceuticals, belebenden Mitteln, lindernden Mitteln, Aminosäuren, chemosthetischen Mitteln (chemsthetic agents), Antioxidantien, Pflanzenstoffen, Zähne-weiß-machenden Mitteln, therapeutischen Mitteln, und Kombinationen davon; oder
(vi) weiterhin umfassend einen Geschmacksstoff, dispergiert in dem Körper oder den ausgelaugten Tabakfasern, derart dass der Geschmacksstoff freigesetzt wird, wenn der Körper innerhalb eines Mundes eines erwachsenen Konsumenten behalten wird.

11. Das orale Produkt nach Anspruch 1(C), wobei
(i) der Körper mindestens zehn Gewichtsprozent ausgelaugte Tabakfasern umfasst;
(ii) der Körper mindestens zehn Gewichtsprozent des Mund-stabilen Polymers umfasst;
(iii) der Körper zwischen 0,1 mg und 6 mg Nikotin umfasst;
(iv) der Körper eine Kompressibilität @ 250 N von zwischen 45% und 90% aufweist;
(v) der Körper eine Kompressibilität @ 425 N von zwischen 60% und 98% aufweist; oder
(vi) der Körper einen Prozentsatz an Elastizität von zwischen 75% und 90% aufweist.

12. Das orale Tabakprodukt nach einem der Ansprüche 1(D)-(F),
(i) wobei die Mund-stabile Polymermatrix Polyurethan umfasst;
(ii) weiterhin umfassend einen Weichmacher, der in der Mund-stabilen Polymermatrix dispergiert ist;
(iii) wobei der Körper schildförmig ist;
(iv) wobei der Körper mindestens zehn Gewichtsprozent an Tabakfasern umfasst;
(v) weiterhin umfassend Nicht-Tabak-Zellulosefasern;
(vi) wobei der Körper mindestens zehn Gewichtsprozent des Mund-stabilen Polymers umfasst;
(vii) wobei der Körper eine Kompressibilität @ 250 N von zwischen 45% und 90% aufweist;
(viii) wobei der Körper eine Kompressibilität @425 N von zwischen 60% und 98% aufweist;
(ix) wobei der Körper einen Prozentsatz an Elastizität von zwischen 75% und 90% aufweist;
(x) weiterhin umfassend ein Antioxidationsmittel, vorzugsweise, wobei das orale Produkt zwischen 0,01 Gewichtsprozent und 5,0 Gewichtsprozent Antioxidationsmittel umfasst; oder
(xi) weiterhin umfassend eine lösliche Faser, vorzugsweise, wobei das Verhältnis der löslichen Faser zu den Zellulosefasern zwischen 1:60 und 60:1 beträgt, oder wobei die löslichen Fasern Maltodextrin umfassen.

13. Das Verfahren nach Anspruch 3(H), wobei das Verfahren weiterhin das Zugeben von löslichen Fasern während des Extrudierschritts umfasst.

## Revendications

1. Produit oral,
(A) comprenant un corps qui peut être entièrement reçu dans une cavité orale, le corps comprenant :
une matrice de polymère stable dans la bouche ;
des fibres cellulosiques incorporées dans la matrice de polymère stable dans la bouche ; et
de la nicotine ou un dérivé de celle-ci dispersé dans la matrice de polymère stable dans la bouche, de telle sorte que la nicotine ou le dérivé de celle-ci est libéré du corps lorsque le corps est reçu dans la cavité orale et exposé à la salive ;
(B) comprenant un corps qui peut être entièrement reçu dans une cavité orale, le corps comprenant :
une matrice de polymère stable dans la bouche ;
des fibres cellulosiques incorporées dans la matrice de polymère stable dans la bouche ; et
un additif dispersé dans la matrice de polymère stable dans la bouche, de telle sorte que l'additif est libéré du corps lorsque le corps est reçu dans la cavité orale et exposé à la salive ;
(C) comprenant un corps qui peut être entièrement reçu dans une cavité orale, le corps comprenant :
une matrice de polymère stable dans la bouche ;
des fibres de tabac épuisé incorporées dans la matrice de polymère stable dans la bouche ; et
un additif dispersé dans la matrice de polymère stable dans la bouche, de telle sorte que l'additif est libéré du corps lorsque le corps est reçu dans la cavité orale et exposé à la salive ;
(D) qui est un produit de tabac oral, comprenant un corps qui peut être entièrement reçu dans une cavité orale, le corps comprenant :
une matrice de polymère stable dans la bouche ;
des fibres de tabac incorporées dans la matrice de polymère stable dans la bouche ;
dans lequel le corps a une compressibilité à 250 N comprise entre 45 % et 95 % ;
(E) qui est un produit de tabac oral, comprenant un corps qui peut être entièrement reçu dans une cavité orale, le corps comprenant :
une matrice de polymère stable dans la bouche ;
des fibres de tabac incorporées dans la matrice de polymère stable dans la bouche ; dans lequel le corps a une compressibilité à 425 N comprise entre 60 % et 99 % ; ou
(F) qui est un produit de tabac oral, comprenant un corps qui peut être entièrement reçu dans une cavité orale, le corps comprenant :
une matrice de polymère stable dans la bouche ;
des fibres de tabac incorporées dans la matrice de polymère stable dans la bouche ;
dans lequel le corps a un pourcentage d'élasticité d'au moins 20 % ;
dans lequel, dans (A) à (F) ci-dessus,
la matrice de polymère stable dans la bouche comprend un polymère choisi dans le groupe constitué par les polyuréthanes, les polymères de silicone, les polyesters, les polyacrylates, les polyéthylènes, les polypropylènes, les polyétheramides, les polystyrènes, les alcools polyvinyliques, les poly(acétate de vinyle), les poly(chlorure de vinyle), les poly(acétate de butyle), les caoutchoucs butyle et les mélanges, les combinaisons et les copolymères de ceux-ci, et dans lequel la matrice de polymère stable dans la bouche ne se dissous pas ou ne se désintègre pas sensiblement lorsqu'elle est exposée à la salive à environ 37 °C (environ 98,6 °F) pendant une période d'une heure ; et
dans lequel les fibres cellulosiques comprennent de la cellulose ou un matériau cellulosique traité.

2. Produit oral selon la revendication 1(A),
(i) dans lequel la matrice de polymère stable dans la bouche comprend un polyuréthane, et dans lequel de préférence, la nicotine est de la nicotine synthétique ;
(ii) dans lequel la matrice de polymère stable dans la bouche comprend un polyester, et dans lequel de préférence, la nicotine est de la nicotine synthétique ;
(iii) dans lequel la matrice de polymère stable dans la bouche comprend un polyacrylate, et dans lequel de préférence, la nicotine est de la nicotine synthétique
(iv) dans lequel la matrice de polymère stable dans la bouche comprend du polyéthylène, du SEBS, du SBS ou une combinaison de ceux-ci, et dans lequel de préférence, la nicotine est de la nicotine synthétique ;
(v) comprenant en outre un plastifiant dispersé dans la matrice de polymère stable dans la bouche, et dans lequel de préférence, la nicotine est de la nicotine synthétique, dans lequel le plastifiant est de préférence choisi dans le groupe constitué par le propylène glycol, la glycérine, une huile végétale, les triglycérides et les combinaisons de ceux-ci ;
(vi) comprenant en outre un édulcorant dispersé dans le corps, et dans lequel de préférence, la nicotine est de la nicotine synthétique, dans lequel l'édulcorant est de préférence choisi dans le groupe constitué par la saccharine, le sucralose, l'aspartame, l'acésulfame de potassium, et les combinaisons de ceux-ci ;
(vii) comprenant en outre un additif choisi dans le groupe comprenant les minéraux, les vitamines, les compléments alimentaires, les nutraceutiques, les agents énergisants, les agents apaisants, les acides aminés, les agents chimiosensoriels, les antioxydants, les plantes, les agents de blanchiment des dents, les agents thérapeutiques et les combinaisons de ceux-ci, dans lequel l'additif est dispersé dans le corps de telle sorte que l'additif est libéré lorsque le corps est maintenu dans la bouche d'un consommateur adulte ;
(viii) comprenant en outre un agent aromatisant dispersé dans le corps de telle sorte que l'agent aromatisant est libéré lorsque le corps est maintenu dans la bouche d'un consommateur adulte, dans lequel l'agent aromatisant est de préférence choisi dans le groupe constitué par la réglisse, les aromatisants de type thé des bois, cerise et baie, Drambuie, bourbon, scotch, whisky, menthe verte, menthe poivrée, lavande, cannelle, cardamome, Apium graveolens, clou de girofle, cascarille, muscade, bois de santal, bergamote, géranium, essence de miel, essence de rose, vanille, essence de citron, essence d'orange, menthe japonaise, cassia, carvi, cognac, jasmin, camomille, menthol, ylang ylang, sauge, fenouil, pimenta, gingembre, anis, coriandre, café, essences de menthe d'une espèce du genre Mentha, et les combinaisons de ceux-ci.
(ix) dans lequel la nicotine est dérivée du tabac ;
(x) dans lequel la nicotine est de la nicotine synthétique ;
(xi) dans lequel le produit oral est sensiblement exempt de tissus végétaux de tabac ;
(xii) dans lequel la nicotine est absorbée dans la matrice de fibre-polymère ;
(xiii) dans lequel le corps est en forme de bouclier ;
(xiv) dans lequel le corps a un diamètre compris entre 5 mm et 25 mm et une épaisseur comprise entre 1 mm et 10 mm ;
(xv) dans lequel le corps comprend au moins 10 pour cent en poids de fibres cellulosiques ;
(xvi) dans lequel les fibres cellulosiques sont des fibres cellulosiques autres que le tabac, de préférence les fibres cellulosiques sont des fibres de betterave à sucre, une fibre de pâte de bois, une fibre de coton, une fibre de son, une fibre de pulpe d'agrumes, une fibre d'herbe, une fibre de saule et une fibre de peuplier ;
(xvii) dans lequel le corps comprend au moins 10 pour cent en poids du polymère stable dans la bouche ;
(xviii) dans lequel le corps comprend entre 0,1 mg et 6 mg de nicotine ;
(xix) dans lequel le corps a une compressibilité à 250 N de moins de 95 % ;
(xx) dans lequel le corps a une compressibilité à 250 N de moins de 80 % ;
(xxi) dans lequel le corps a une compressibilité à 250 N comprise entre 45 % et 90 % ;
(xxii) dans lequel le corps a une compressibilité à 425 N de moins de 99 % ; le corps a une compressibilité à 425 N comprise entre 60 % et 98 % ;
(xxiii) dans lequel le corps a un pourcentage d'élasticité d'au moins 20 % ;
(xxiv) dans lequel le corps a un pourcentage d'élasticité d'au moins 70 % ;
(xxv) dans lequel le corps a un pourcentage d'élasticité compris entre 75 % et 90 % ;
(xxvi) dans lequel le produit oral comprend en outre un antioxydant ; dans lequel le produit oral comprend de préférence entre 0,01 pour cent en poids et 5,0 pour cent en poids d'antioxydant ; ou dans lequel l'antioxydant est de préférence choisi parmi le palmitate d'ascorbyle, le BHT, l'acide ascorbique, l'ascorbate de sodium, le citrate de monostérol, les tocophérols, le gallate de propyle, la butylhydroquinone tertiaire (TBHQ), l'hydroxyanisole butylé (BHA), la vitamine E, et une combinaison de ceux-ci ; ou dans lequel l'antioxydant est un dérivé de la vitamine C ; ou dans lequel l'antioxydant est le palmitate d'ascorbyle ; ou (xxvii) comprenant en outre un antioxydant et une fibre soluble, dans lequel le rapport des fibres solubles aux fibres cellulosiques est de préférence compris entre 1:60 et 60:1 ; ou dans lequel les fibres solubles comprennent de la maltodextrine ; ou dans lequel les fibres solubles sont dérivées du maïs.

3. Procédé de formation d'un produit oral selon la revendication 1, comprenant :
(A) l'extrusion d'un polymère stable dans la bouche ayant des fibres cellulosiques dispersées dans celui-ci ; et la dispersion de nicotine ou d'un dérivé de celle-ci dans le polymère stable dans la bouche pendant ou après l'étape d'extrusion ;
(B) l'extrusion d'un polymère stable dans la bouche ;
la dispersion de nicotine dans le polymère stable dans la bouche pendant ou après l'étape d'extrusion ; et
la découpe du polymère stable dans la bouche extrudé pour former des produits oraux individuels ;
(C) l'extrusion d'un polymère stable dans la bouche ;
la dispersion de nicotine dans le polymère stable dans la bouche pendant ou après l'étape d'extrusion ; et
le façonnage du polymère stable dans la bouche extrudé pour former des produits oraux individuels ;
(D) l'extrusion d'un polymère stable dans la bouche ayant des fibres cellulosiques dispersées dans celui-ci ; et
la dispersion d'un additif dans le polymère stable dans la bouche pendant ou après l'étape d'extrusion, l'additif étant choisi parmi un agent aromatisant, un édulcorant, la caféine ou une combinaison de ceux-ci ;
(E) l'extrusion d'un polymère stable dans la bouche ;
la dispersion d'un additif dans le polymère stable dans la bouche pendant ou après l'étape d'extrusion ; et
la découpe du polymère stable dans la bouche extrudé pour former des produits oraux individuels ;
(F) l'extrusion d'un polymère stable dans la bouche ;
la dispersion d'un additif dans le polymère stable dans la bouche pendant ou après l'étape d'extrusion ; et
le façonnage du polymère stable dans la bouche extrudé pour former des produits oraux individuels ;
(G) l'extrusion d'un polymère stable dans la bouche ayant des fibres de tabac épuisé dispersées dans celui-ci ; et
la dispersion d'un additif dans le polymère stable dans la bouche pendant ou après l'étape d'extrusion, l'additif étant choisi parmi un agent aromatisant, un édulcorant, la nicotine, la caféine ou une combinaison de ceux-ci ; ou
(H) dans lequel le produit oral est un produit de tabac oral et le procédé comprend :
l'extrusion d'un polymère stable dans la bouche ayant des fibres de tabac épuisé dispersées dans celui-ci ; et
la découpe du mélange extrudé de polymère stable dans la bouche et de fibres de tabac en produits de tabac oraux individuels dimensionnés de manière à être au moins partiellement reçus dans la cavité orale d'un consommateur de tabac adulte.

4. Procédé selon la revendication 3(A),
(i) comprenant en outre la découpe du mélange extrudé de polymère stable dans la bouche et de fibres cellulosiques en produits oraux individuels dimensionnés de manière à être au moins partiellement reçus dans la cavité orale d'un consommateur adulte ;
(ii) dans lequel l'étape de dispersion a lieu pendant l'étape d'extrusion, dans lequel la nicotine est de préférence mélangée avec les fibres cellulosiques ;
(iii) dans lequel l'étape de dispersion a lieu après l'étape d'extrusion ;
(iv) dans lequel le polymère stable dans la bouche comprend un plastifiant mélangé dans celui-ci pendant l'étape d'extrusion ;
(v) dans lequel le procédé comprend en outre l'ajout d'un agent aromatisant pendant ou après l'étape d'extrusion ;
(vi) dans lequel le procédé comprend en outre l'ajout d'un antioxydant pendant l'étape d'extrusion ; ou
(vii) dans lequel le procédé comprend en outre l'ajout d'un antioxydant pendant un processus d'aromatisation post-extrusion ; ou
(viii) dans lequel le procédé comprend en outre l'ajout de fibres solubles pendant l'étape d'extrusion.

5. Procédé selon la revendication 3(B), dans lequel les produits oraux individuels sont souples et ont une mémoire de forme lorsqu'ils sont manipulés à l'intérieur d'une cavité orale.

6. Produit oral selon la revendication 1(A), dans lequel
(i) le corps comprend entre 0,01 pour cent en poids et 1,0 pour cent en poids d'antioxydant et entre 0,1 mg et 6 mg de nicotine ;
(ii) un antioxydant est dispersé dans le corps, dans lequel l'antioxydant est le palmitate d'ascorbyle, le BHT, l'acide ascorbique, l'ascorbate de sodium, un dérivé de la vitamine C, le citrate de monostérol, les tocophérols, le gallate de propyle, la butylhydroquinone tertiaire (TBHQ), l'hydroxyanisole butylé (BHA), la vitamine E, et un dérivé ou une combinaison de ceux-ci ; ou
(iii) le corps comprend une combinaison de fibres solubles et de fibres cellulosiques insolubles dans la matrice, dans lequel la nicotine et l'antioxydant sont dispersés dans la matrice.

7. Produit oral selon la revendication 1(B),
(i) dans lequel la matrice de polymère stable dans la bouche comprend un polyuréthane ;
(ii) comprenant en outre un édulcorant dispersé dans le corps ;
(iii) dans lequel l'additif est choisi dans le groupe constitué par les minéraux, les vitamines, les compléments alimentaires, les nutraceutiques, les agents énergisants, les agents apaisants, les acides aminés, les agents chimiosensoriels, les antioxydants, les plantes, les agents de blanchiment des dents, les agents thérapeutiques et les combinaisons de ceux-ci,
(iv) dans lequel l'additif est un agent thérapeutique choisi dans le groupe constitué par le Gerd, la buprénorphine, la nitroglycérine, le diclofénac, le fentanyl, la carbamazépine, la galantamine, l'acyclovir, des nanoparticules de polyamidoamine, la chlorphéniramine, la testostérone, l'oestradiol, la progestérone, la calcitonine, le fluorouracile, la naltrexone, l'odansétron, la décitabine, la sélégiline, la lamotrigine, la prochlorpérazine, et les combinaisons de ceux-ci ;
(V) comprenant en outre un agent aromatisant dispersé dans le corps de telle sorte que l'agent aromatisant est libéré lorsque le corps est maintenu dans la bouche d'un consommateur ;
(vi) dans lequel le corps a une compressibilité à 250 N comprise entre 45 % et 90 % ;
(vii) dans lequel le corps a une compressibilité à 425 N comprise entre 60 % et 98 % ; ou
(viii) dans lequel le corps a un pourcentage d'élasticité compris entre 75 % et 90 %.

8. Procédé selon la revendication 3(E), dans lequel les produits oraux individuels ont une compressibilité à 250 N comprise entre 45 % et 90 %.

9. Procédé selon la revendication 3(F), dans lequel les produits oraux individuels ont une compressibilité à 250 N comprise entre 45 % et 90 %.

10. Produit oral selon la revendication 1(C),
(i) dans lequel la matrice de polymère stable dans la bouche comprend un polyuréthane ;
(ii) comprenant en outre un édulcorant dispersé dans le corps ;
(iii) dans lequel l'additif est la nicotine ou un dérivé de celle-ci ;
(iv) dans lequel le produit oral comprend en outre des fibres cellulosiques autres que le tabac ;
(v) dans lequel l'additif est choisi dans le groupe constitué par les minéraux, les vitamines, les compléments alimentaires, les nutraceutiques, les agents énergisants, les agents apaisants, les acides aminés, les agents chimiosensoriels, les antioxydants, les plantes, les agents de blanchiment des dents, les agents thérapeutiques et les combinaisons de ceux-ci ; ou
(vi) comprenant en outre un agent aromatisant dispersé dans le corps ou les fibres de tabac épuisé de telle sorte que l'agent aromatisant est libéré lorsque le corps est maintenu dans la bouche d'un consommateur adulte.

11. Produit oral selon la revendication 1(C), dans lequel
(i) le corps comprend au moins 10 pour cent en poids de fibres de tabac épuisé ;
(ii) le corps comprend au moins 10 pour cent en poids du polymère stable dans la bouche ;
(iii) le corps comprend entre 0,1 mg et 6 mg de nicotine ;
(iv) le corps a une compressibilité à 250 N comprise entre 45 % et 90 % ;
(v) le corps a une compressibilité à 425 N comprise entre 60 % et 98 % ; ou
(vi) le corps a un pourcentage d'élasticité compris entre 75 % et 90 %.

12. Produit de tabac oral selon l'une quelconque des revendications 1(D) à (F),
(i) dans lequel la matrice de polymère stable dans la bouche comprend un polyuréthane ;
(ii) comprenant en outre un plastifiant dispersé dans la matrice de polymère stable dans la bouche ;
(iii) dans lequel le corps est en forme de bouclier ;
(iv) dans lequel le corps comprend au moins 10 pour cent en poids de fibres de tabac ;
(v) comprenant en outre des fibres cellulosiques autres que le tabac ;
(vi) dans lequel le corps comprend au moins 10 pour cent en poids du polymère stable dans la bouche ;
(vii) dans lequel le corps a une compressibilité à 250 N comprise entre 45 % et 90 % ;
(viii) dans lequel le corps a une compressibilité à 425 N comprise entre 60 % et 98 % ;
(ix) dans lequel le corps a un pourcentage d'élasticité compris entre 75 % et 90 % ;
(x) comprenant en outre un antioxydant, de préférence, dans lequel le produit oral comprend entre 0,01 pour cent en poids et 5,0 pour cent en poids d'antioxydant ; ou
(xi) comprenant en outre une fibre soluble, de préférence, dans lequel le rapport des fibres solubles aux fibres cellulosiques est compris entre 1:60 et 60:1, ou dans lequel les fibres solubles comprennent de la maltodextrine.

13. Procédé selon la revendication 3(H), dans lequel le procédé comprend en outre l'ajout de fibres solubles pendant l'étape d'extrusion.
